# EUROPEAN PATENT SPECIFICATION

(11) **EP 4 696 219 B1**
(45) Date of publication and mention of the grant of the patent: **30.09.2026**
(21) Application number: 24194535.1
(22) Date of filing: 14.08.2024
(51) Int. Cl.: A61B 3/10

(54) **PATIENT ALIGNMENT SYSTEM FOR AN OPHTHALMIC IMAGING INSTRUMENT**
PATIENTENAUSRICHTUNGSSYSTEM FÜR EIN OPHTHALMISCHES BILDGEBUNGSINSTRUMENT
SYSTÈME D'ALIGNEMENT DE PATIENT POUR UN INSTRUMENT D'IMAGERIE OPHTALMIQUE

(43) Date of publication of application: 18.02.2026
(73) Proprietor: Optos plc, Dunfermline, Scotland KY11 8GR (GB)
(72) Inventor: SWAN, Derek, Dunfermline, Scotland KY11 8GR (GB); PEMBERTON, Stephen, Dunfermline, Scotland KY11 8GR (GB)
(74) Representative: Hoffmann Eitle

(56) References cited:
- US-A1- 2016 089 024
- US-A1- 2017 347 882
- US-A1- 2020 237 217

## Description

### [Field]

Example aspects herein generally relate to the field of ophthalmic imaging scanners and, more particularly, to systems for bringing an eye of a subject into a position for acquiring an image of a fundus of the eye by an ophthalmic imaging scanner.

### [Background]

Ophthalmic imaging scanners employ various techniques to image different parts of an eye of a subject, and are used by clinicians to diagnose and manage a variety of eye conditions. Ophthalmic imaging scanners acquire images of the fundus or other part of the eye by scanning a beam of light across the eye and detecting return light from the eye. To acquire high quality images of the fundus of the eye, ophthalmic imaging scanners, such as scanning laser ophthalmoscopes (SLOs) and optical coherence tomography (OCT) scanners, for example, often require the position of an exit pupil of the ophthalmic imaging scanner to fall within a predetermined range of distances from the pupil of the eye that are suitable for acquiring such images. Pupil alignment can be particularly important for widefield (WF) and ultra-widefield (UWF) ophthalmic imaging scanners, where the acquisition of a WF or UWF fundus image relies on light propagating to and from the fundus, through the pupil, at widely varying angles of incidence on the pupillary plane.

For example, US 2020/237217 A1 discloses an ophthalmic device including an illumination module which scans light across a region of the retina of an eye when the pupil is disposed at a focal point of the illumination module. The ophthalmic device further comprises components for: aligning the pupil with the focal point; monitoring a position of the pupil relative to the focal point and maintaining the alignment based on the monitored position; aligning a scan location of the illumination module on the retina to a target scan location while the alignment of the pupil with the focal point is being maintained, wherein the illumination module performs scan at the target scan location The ophthalmic device further maintains the scan location at the target scan location while the alignment of the pupil with the focal point is being maintained, using scan location correction information based on retinal feature information.

Stereoscopic ranging techniques employing stereo cameras are often used to measure distance to the eye. For example, some ophthalmic imaging scanners include a so-called pupil alignment module (PAM), which comprises stereo cameras to acquire stereo images of the eye, and is arranged to locate respective pupil centres in the stereo images, and determine the distance between the PAM and the pupil based on a separation between the located pupil centres in the stereo images. Visual feedback based on the determined distance is provided to guide the subject in bringing their eye into a position suitable for imaging the eye.

The stereo cameras of a PAM are typically mounted on the ophthalmic imaging scanner to have a direct visual pathway to the subject's eye (i.e. with there being no intervening optical element). However, in a WF or UWF ophthalmic imaging scanner, the mirror based WF/UWF optics that are often used for the acquisition of the WF or UWF image, which typically employing an ellipsoidal mirror to transfer the scan to/from the eye, can make the provision of such a direct visual pathway difficult or impossible. The alternative of guiding light from the eye to the stereo cameras using additional optical elements within the WF/UWF optics can increase the complexity of the system and present significant engineering challenges, for example in dealing with spurious reflections, which may be caused by the additional optical elements, and which may manifest as artifacts in the acquired images.

It would therefore be desirable to find a way of accommodating a stereo imaging apparatus for acquiring stereo imaging of the pupil within a mirror based WF or UWF ophthalmic imaging scanner that at least partially addresses the problems set out above.

### [Summary]

The present invention is defined in independent claim 1, directed to an ultra-widefield ophthalmic imaging instrument, and in independent claim 14, directed to a corresponding method of controlling such an instrument. Preferred embodiments of the invention are set forth in dependent claims 2-13 and 15 respectively.

There is provided, in accordance with a first embodiment herein, an ultra-widefield (UWF) ophthalmic imaging instrument arranged to acquire an UWF image of a fundus of an eye of a subject by scanning a beam of light across the fundus, comprising: a light source arranged to emit the beam of light; a rotatable mirror arranged to scan the beam of light across the fundus; and an ellipsoidal mirror via which the rotatable mirror is arranged to scan the beam of light across the fundus, the ellipsoidal mirror having a first focal point and a second focal point, wherein the rotatable mirror is arranged to scan the beam of light across the ellipsoidal mirror via the first focal point, and a pupil of the eye is positioned at the second focal point during use of the ultra-widefield ophthalmic imaging instrument. The UWF ophthalmic imaging instrument further comprises a stereo imaging apparatus arranged to acquire stereo images of the pupil via the rotatable mirror and the ellipsoidal mirror; and a processor arranged to operate in a patient alignment mode to generate, based on the stereo images of the pupil, a signal for bringing the pupil of the eye within a target range for acquiring an UWF image of the fundus, and to subsequently operate in a fundus imaging mode to control the ultra-widefield ophthalmic imaging instrument to acquire the UWF image of the fundus. In the patient alignment mode, the processor is arranged to: control the rotatable mirror to be stationary in a predetermined orientation; control the stereo imaging apparatus to acquire the stereo images of the pupil via the rotatable mirror and the ellipsoidal mirror while the rotatable mirror is stationary in the predetermined orientation; and process the stereo images of the pupil to generate the signal for bringing the pupil of the eye into alignment with the imaging position. In the fundus imaging mode, the processor is arranged to: control the light source to emit the beam of light; and control the ultra-widefield ophthalmic imaging instrument to acquire the UWF image of the fundus by controlling the rotatable mirror to scan the beam of light across the fundus via the ellipsoidal mirror.

In some example embodiments, the rotatable mirror is arranged to reflect the beam of light across the ellipsoidal mirror at the first focal point. In other example embodiments, the UWF ophthalmic imaging instrument further comprises a second ellipsoidal mirror, via which the rotatable mirror is arranged to scan the beam of light across the fundus, the second ellipsoidal mirror having a first focal point and a second focal point, wherein the rotatable mirror is arranged to reflect the beam of light at the first focal point of the second ellipsoidal mirror, and the second focal point of the second ellipsoidal mirror coincides with the first focal point of the ellipsoidal mirror.

In example embodiments where the rotatable mirror is arranged to reflect the beam of light across the ellipsoidal mirror at the first focal point of the ellipsoidal mirror, the stereo imaging apparatus may be located within a region between the rotatable mirror and the ellipsoidal mirror not traversed by the beam of light during acquisition of the UWF image of the fundus. In the example embodiments comprising the second ellipsoidal mirror noted above, the stereo imaging apparatus may be located within a region between the rotatable mirror and the second ellipsoidal mirror not traversed by the beam of light during acquisition of the UWF image of the fundus.

The predetermined orientation of the rotatable mirror may be such that the rotatable mirror starts rotating from the predetermined orientation when the ultra-widefield ophthalmic imaging instrument starts acquiring the UWF image of the fundus.

Additionally or alternatively, the stereo imaging apparatus may be located between the rotatable mirror and the ellipsoidal mirror at a location which minimises a deviation from circularity of respective representations of the pupil in the stereo images of the pupil acquired by the stereo imaging apparatus when the rotatable mirror is in the predetermined orientation. In some example embodiments, the ellipsoidal mirror may have a plane of symmetry comprising the first focal point and the second focal point, a normal direction of the rotatable mirror at the first focal point may subtend a predetermined angle relative to the plane of symmetry when the rotatable mirror is in the predetermined orientation, and the stereo imaging apparatus may comprise a first camera and a second camera, wherein the first camera and the second camera are arranged equidistantly from and on opposite sides of a second plane comprising the first focal point and the second focal point, and wherein the normal direction of the rotatable mirror and the second plane subtend an angle at the first focal point which is less than two times as large as the predetermined angle. In these example embodiments, the first camera may have a first optical axis, and the second camera may have a second optical axis which is parallel to the first optical axis, and the stereo imaging apparatus may further comprise a Fresnel lens disposed between the rotatable mirror and both the first camera and the second camera, the Fresnel lens being arranged to refract light from the eye, which has been reflected by the rotatable mirror (e.g. at the first focal point of the ellipsoidal mirror), to propagate along the first optical axis and the second optical axis. In some other example embodiments, the stereo imaging apparatus may comprise a first camera having a first optical axis, a second camera having a second optical axis which is parallel to the first optical axis, and a Fresnel lens disposed between the rotatable mirror and both the first camera and the second camera, the Fresnel lens being arranged to refract light from the eye, which has been reflected by the rotatable mirror (e.g. at the first focal point of the ellipsoidal mirror, in case the rotatable mirror is arranged to reflect the beam of light at the first focal point), to propagate along the first optical axis and the second optical axis.

The UWF ophthalmic imaging instrument of the first embodiment or any of its example embodiments set out above may further comprise a fixation target light source arranged to project fixation light onto the fundus via the rotatable mirror and the ellipsoidal mirror and, in the patient alignment mode, the processor may be arranged to control the fixation target light source to project the fixation light onto the rotatable mirror in the predetermined orientation, the fixation target light source being disposed in relation to the rotatable mirror such that the fixation light is projected onto the fundus via the ellipsoidal mirror to fix a gaze direction of the eye. In some example embodiments, wherein the rotatable mirror is arranged to reflect the beam of light at the first focal point, and the stereo imaging apparatus is located within the region between the rotatable mirror and the ellipsoidal mirror not traversed by the beam of light during acquisition of the UWF image of the fundus, the fixation target light source may also located within that region. In this case, the predetermined orientation of the rotational mirror may be such that the rotatable mirror starts rotating from the predetermined orientation when the ultra-widefield ophthalmic imaging instrument starts acquiring the UWF image of the fundus. For example, the fixation target light source may be arranged to project the fixation light onto the rotatable mirror from a location such that, when the rotatable mirror is in the predetermined orientation, the fixation light is incident on the eye in a direction for central gaze fixation of the eye.

In the UWF ophthalmic imaging instrument of the first embodiment or any of its example embodiments set out above, the signal for bringing the pupil of the eye within the target range for acquiring the UWF image of the fundus may comprise: a projection of light of a first colour (e.g. blue) onto the eye in case the subject is to move the eye towards the UWF ophthalmic imaging instrument to bring the pupil towards the target range; a projection of light of a second colour (e.g. red) onto the eye in case the subject is to move the eye away from the UWF ophthalmic imaging instrument to bring the pupil towards the target range; and a projection of light of a third colour (e.g. green) onto the eye in case the pupil is within the target range suitable for acquiring the UWF image, wherein the first colour, the second colour and the third colour are different from one another. Additionally or alternatively, the signal for bringing the pupil of the eye within the target range for acquiring the UWF image of the fundus may comprise: a first sound (e.g. tone or spoken word(s)) in case the subject is to move the eye towards the UWF ophthalmic imaging instrument to bring the pupil towards the target range; a second sound (e.g. tone or spoken word(s)) in case the subject is to move the eye away from the UWF ophthalmic imaging instrument to bring the pupil towards the target range; and a third sound (e.g. tone or spoken word(s)) in case the pupil is within the target range suitable for acquiring the UWF image, wherein the first sound, the second sound and the third sound are different from one another.

The UWF ophthalmic imaging instrument of the first embodiment or any of its example embodiments set out above may comprise an illumination source arranged to illuminate the eye via the rotatable mirror and the ellipsoidal mirror with light for acquiring the stereo images of the pupil by the stereo imaging apparatus, and may comprise an ultra-widefield scanning laser ophthalmoscope, for example.

There is provided, in accordance with a second embodiment herein, a method of controlling an UWF ophthalmic imaging instrument to acquire an UWF image of a fundus of an eye by scanning a beam of light across the fundus, the ultra-widefield ophthalmic imaging instrument comprising: a light source arranged to emit the beam of light; a rotatable mirror arranged to scan the beam of light across the fundus; an ellipsoidal mirror via which the rotatable mirror is arranged to scan the beam of light across the fundus, the ellipsoidal mirror having a first focal point and a second focal point, wherein the rotatable mirror is arranged to scan the beam of light across the ellipsoidal mirror via the first focal point and a pupil of the eye is positioned at the second focal point during use of the ultra-widefield ophthalmic imaging instrument; and a stereo imaging apparatus arranged to acquire stereo images of the pupil via the rotatable mirror and the ellipsoidal mirror. The method comprises generating a signal to bring the pupil of the eye within a target range for acquiring the UWF image of the fundus, by: controlling the rotatable mirror to be stationary in a predetermined orientation that allows the stereo imaging apparatus to image the pupil via the rotatable mirror and the ellipsoidal mirror; controlling the stereo imaging apparatus to acquire the stereo images of the pupil via the rotatable mirror and the ellipsoidal mirror while the rotatable mirror is in the predetermined orientation; and processing the stereo images of the pupil to generate the signal. The method further comprises, after generating the signal, controlling the light source to emit the beam of light, and controlling the ultra-widefield ophthalmic imaging instrument to acquire the UWF image of the fundus by rotating the rotatable mirror to scan the beam of light across the fundus via the ellipsoidal mirror.

In some example embodiments, the UWF ophthalmic imaging instrument may further comprise a fixation target light source arranged to project fixation light onto the fundus via the rotatable mirror and the ellipsoidal mirror, and the method may further comprise, whilst the rotatable mirror is in the predetermined orientation and the stereo imaging apparatus is being controlled to acquire the stereo images of the pupil, controlling the fixation target light source to project the fixation light onto the rotatable mirror, the fixation target light source being disposed in relation to the rotatable mirror such that the fixation light is projected onto the fundus via the ellipsoidal mirror to fix a gaze direction of the eye.

There is also provided, in accordance with a third example herein, a computer program comprising computer-readable instructions that, when executed by the processor of the UWF ophthalmic imaging instrument of the first example or any of its example embodiments set out above, cause the processor to perform the method of the second example or any of its embodiments set out above. The computer program may be stored on a non-transitory computer-readable storage medium (such as a computer hard disk or a CD, for example) or carried by a computer-readable signal.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of an ultra-widefield ophthalmic imaging instrument according to an example embodiment herein.
Figure 2 is a schematic illustration of a programmable signal processing hardware, which may be configured to perform the functions of the processor 50 described herein.
Figure 3 is schematic showing a cross-section of the ellipsoidal mirror 32 of the example embodiment in a plane which is perpendicular to the axis of symmetry of the ellipsoidal mirror and includes the first focal point 32-1, as well as top view of the stereo imaging apparatus 40 and fixation target light source 80 of the example embodiment, along the axis of symmetry.
Figure 4 is a schematic illustration of an ultra-widefield ophthalmic imaging instrument according to a variant of the example embodiment herein, which comprises two ellipsoidal mirrors having a shared focal point, wherein the rotating mirror 30 is provided at the remaining focal point of one of the ellipsoidal mirrors to scan line-field illumination across the fundus 12 via the pupil 14 provided at the remaining focal point of the other ellipsoidal mirror.
Figure 5 is a schematic illustration of a pupil alignment module (PAM) of an UWF ophthalmic imaging instrument of an example embodiment, when viewed from the rotatable mirror 30. The PAM comprises the stereo imaging system 40 and a modified version of the fixation target light source 80 included in the example embodiment of Figure 1.
Figure 6 is a flow diagram illustrating a method by which the processor 50 controls the UWF ophthalmic imaging instrument of the example embodiment to acquire an UWF image 55 of the fundus 12 of the eye 10.

### [Detailed Description of Example Embodiments]

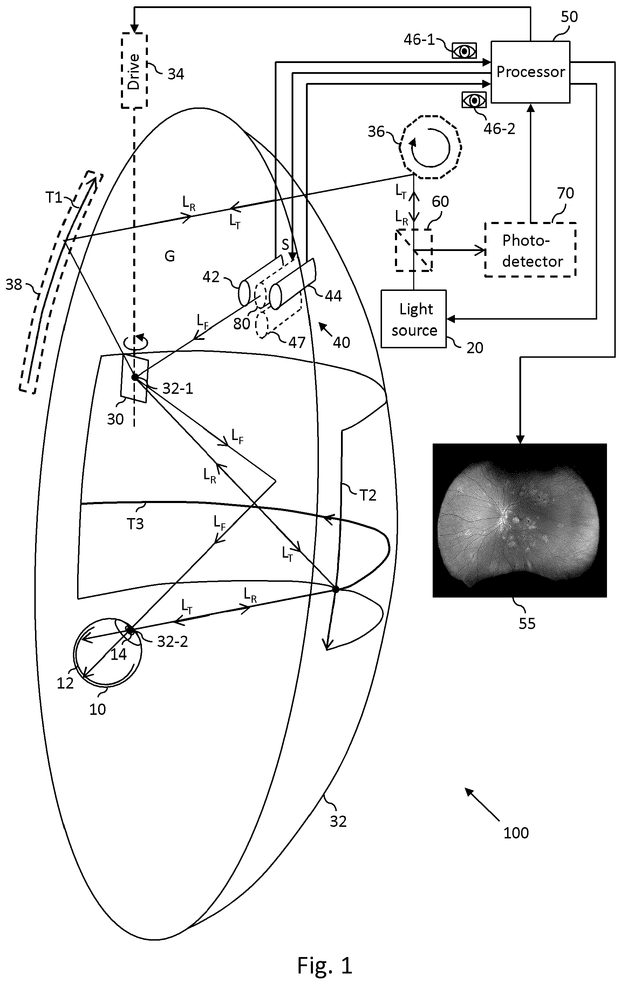

Figure 1 is a schematic illustration of an ophthalmic imaging instrument 100 according to the first example embodiment, which is operable to image a portion of an eye 10 of a subject by scanning a beam of light across the eye 10. The portion imaged may, as in the present example embodiment, comprise a region of a fundus 12 of the eye 10. The ocular fundus 12 is the inner lining of the eye 10 opposite the lens, and comprises the retina, macula, optic disc, fovea, choroid, and blood vessels. The ophthalmic imaging instrument 100 may additionally be operable to image another portion of the eye 10, such as at least a part of the anterior segment of the eye 10.

The ophthalmic imaging instrument 100 may be a widefield (WF) ophthalmic imaging instrument, which is operable to acquire a WF image of the fundus 12. A WF image of the fundus 12 is defined as a single-capture image, centred on the fovea of the eye 10, which captures retinal anatomic features in the mid-periphery that are posterior to the vortex vein ampulla, in all four quadrants (i.e. superior, inferior, nasal and temporal) of the eye 10. A WF ophthalmic imaging instrument can thus acquire a single-capture image which covers a region of the retina extending from the fovea up to the posterior edge of vortex vein ampulla, and is defined to have a field of view (FoV) between 60 and 100 degrees.

Here, the FoV is expressed in terms of the eye-angle, which is the angle subtended by the imaged retinal region at the spherical centre of the eye 10, i.e. the point of intersection between the vertical diameter of the eyeball and the visual axis.

The ophthalmic imaging instrument 100 may, as in the present example embodiment, be provided in form of an ultra-widefield (UWF) ophthalmic imaging instrument, which has a larger FoV than a WF instrument and is operable to acquire an UWF image of the ocular fundus 12 covering a larger proportion of the ocular fundus 12. An UWF image of the ocular fundus 12 is defined as a single-capture image, centred on the fovea of the eye 10, which captures retinal anatomic features in the far periphery that are anterior to the vortex vein ampulla in all four quadrants. An UWF ophthalmic imaging instrument can thus acquire an UWF image being a single-capture image which covers a region of the retina extending from the fovea to the anterior edge of vortex vein ampulla and beyond to pars plana, and is defined to have a FoV (expressed in terms of the eye-angle) between 110 degrees and 220 degrees. By way of an example, the Optos Daytona^{™} is capable of UWF images (so -called Optomap^{™} images) covering up to 200 degrees of the fundus (or approximately 82% of the retina) in a single capture.

It should be noted that the techniques described herein are not limited in their applicability to WF and UWF ophthalmic imaging instruments but are also applicable to ophthalmic imaging instruments having a smaller FoV, and may benefit any scanning ophthalmic imaging instrument which scans light across a portion of the eye 10 via one or more ellipsoidal mirrors to image the portion.

In the present example embodiment, the ophthalmic imaging instrument 100 comprises an UWF combined scanning laser ophthalmoscope (SLO) and optical coherence tomography (OCT) instrument, which is arranged to acquire OCT images of the ocular fundus 12 using well-known interferometric imaging techniques, as well as fundus images of one or more additional modalities. Examples of such additional imaging modalities include pseudo-colour imaging, fundus autofluorescence (FAF), fluorescein angiography (FA), and indocyanine green angiography (ICGA). By way of an example, the Optos Monaco^{™} is a combined SLO-OCT system capable of acquiring green or red (or combined green and red) laser views, and green laser autofluorescence views, as well as OCT views. However, the ophthalmic imaging instrument 100 need not be a combined SLO-OCT system, and may instead be an OCT imaging instrument operable to acquire OCT images only, or an UWF SLO operable in one or more of the aforementioned (or other) SLO imaging modalities but not in an OCT modality. The Optos Daytona^{™} is an example of such a multi-modal UWF SLO.

As illustrated schematically in Figure 1, the ophthalmic imaging instrument 100 comprises a light source 20 arranged to emit at least one beam (understood to be any directional projection) of light L_{T} for imaging the eye 10, and an optical system comprising a rotatable mirror 30 and an ellipsoidal mirror 32. A stereo imaging apparatus 40 (described in more detail below) is provided to assist in bringing the eye 10 into a position at which it can be imaged by the ophthalmic imaging instrument 100. The optical system is arranged to scan the at least one beam of light L_{T} across the eye 10, and collect and guide return light L_{R} from the region of the eye 10 illuminated by the beam of light L_{T}. The return light L_{R} may be a portion of the beam of light L_{T} which has been reflected or scattered from the eye 10, or it may be light stimulated by the beam of light L_{T}, as in the case of the FAF and ICGA imaging modalities, for example.

The ophthalmic imaging instrument 100 further comprises a processor (or controller) 50, and may, as in the present example embodiment, also include a beam splitter 60 and a photodetector 70. The beam splitter 60 is arranged to split off a portion of the return light L_{R} and direct the split-off portion of the return light L_{R} to the photodetector 70. An UWF image 55 of the eye 10 is acquired by the ophthalmic imaging instrument 100 (for example, by the processor 50) by processing the output of the photodetector 70 using well-known techniques.

The optical system may also include a drive mechanism 34 comprising a galvanometer, for example, which is controlled by the processor 50 to cause the rotatable mirror 30 to scan the beam of light L_{T} by undergoing a predetermined rotational motion. This may, as in the present example embodiment, comprise a predetermined oscillatory rotational motion, wherein the rotatable mirror 30 rotates alternatively clockwise and anticlockwise by a predetermined angle (or, in other words, wherein the rotatable mirror 30 repeatedly rotates across an angular range defined about the mirror's axis of rotation, reversing its direction of rotation at each end of the angular range).

The light source 20 is, in general, arranged to generate light in one or more ranges of wavelength that are suitable for imaging the ocular fundus 12 (or other part of the eye 10, as the case may be), for example in the visible spectrum (e.g. red and green light) and/or the near-infrared spectrum. The light source 20 may, for example, comprise one or more laser diodes or one or more super-luminescent diodes (or a combination of one or more laser diodes and one or more super-luminescent diodes), and may also have one or more optical components, such as collimators, apertures and lenses, which are arranged to generate one or more light beams. The optical system (discussed further below) may be arranged to illuminate a region of the ocular fundus 12 with a (flying) spot of light or a line of light (in case of the ophthalmic imaging instrument 100 being a line-field (i.e. line-scanning) system) generated using a cylindrical lens or other well-known components or optical assemblies for generating line-field illumination.

In example embodiments like the present, wherein the ophthalmic imaging instrument 100 is operable in an OCT imaging mode, a swept light source (in case of the ophthalmic imaging instrument 100 being a swept-source OCT (SS-OCT) system) or a broadband source (in case of the ophthalmic imaging instrument 100 being a spectral-domain OCT (SD-OCT) system) may be provided for OCT imaging.

The form of the photodetector 70 is not limited and the photodetector 70 may, for example, comprise a balanced photodetector arrangement comprising two reverse-biased photodiodes whose output photocurrents are subtracted from one another, the subtracted current signal being converted to a voltage detection signal by a transimpedance amplifier. In example embodiments where the ophthalmic imaging instrument 100 is provided in the form of a line-scan SLO, the photodetector 70 may comprise a one- or two-dimensional array of photo-sensing elements. In example embodiments like the present, where the ophthalmic imaging instrument 100 features an OCT imaging modality, a spectrometer (where a SD-OCT set-up is used) or a photodiode detector (where a SS-OCT set-up is used) may be provided to detect interference light from the sample arm and reference arm of the interferometer.

The optical system of the present example embodiment is arranged to perform a two-dimensional point scan of the light L_{T} from the light source 20 across the region of the ocular fundus 12 that is illuminated by the point-scan, and to collect light from the illuminated region during the point-scan. The optical system is arranged to perform the two-dimensional point scan in part by the rotatable mirror 30 scanning the beam of light L_{T} across the ocular fundus 12 via the ellipsoidal mirror 32. Furthermore, the optical system further comprises a scanner 36 and a curved mirror 38, which are arranged to scan the beam of light L_{T} in a first direction across the ellipsoidal mirror 32 via the rotatable mirror 30. The scanner 36 may, as in the present example embodiment, be provided in the form of a polygon scanner comprising a plurality (e.g. 16) facets arranged around the circumference of a wheel which is rotated at a high speed (typically over 30,000 revolutions per minute) to perform high-frequency repeat scans of the light beam L_{T}. However, the scanner 36 may be provided in other forms, such as a galvanometer scanner, a micro-electromechanical system (MEMS) scanning mirror or a resonant scanning mirror, for example, or may be replaced by a cylindrical lens or other means of generating a beam comprising a "fan" of light rays whose projection onto a flat surface forms a line of light in case the optical system is a line-field system. Where the ophthalmic imaging instrument 100 has an OCT imaging modality, as in the present example embodiment, a second galvanometer scanner (not shown in Fig. 1) may be provided to scan the OCT sample beam in the first direction across the ellipsoidal mirror 32, via the rotatable mirror 30.

The light beam L_{T} is reflected, in sequence, by the scanner 36, the curved mirror 38, the rotatable mirror 30 and the ellipsoidal mirror 32, before being incident on the fundus 12 via the pupil 14 of the eye 10. Light from the illuminated region of the ocular fundus 12 follows the same optical path through the optical system as the light beam that had entered the optical system but does so in reverse order, and a part thereof is directed to the photodetector 70 by the beam splitter 60.

The two-dimensional point scan is performed by the scanner 36 scanning the light beam L_{T} in a first (e.g. vertical) direction across the region of the ocular fundus 12, and by the rotatable mirror 30 rotating about its rotational axis to scan the light beam L_{T} in a second (e.g. horizontal) direction across the region of the ocular fundus 12 (which second direction may, as in the present example embodiment, be orthogonal to the first direction). The ellipsoidal mirror 32 has a first focal point 32-1 and a conjugate second focal point 32-2 and may, as in the present example embodiment, take the form of a spheroidal mirror having an axis of rotational symmetry, specifically rotational symmetry about its major axis which passes through the focal points. More generally, any form of curved mirror having a curvature that enables it to transfer light rays incident on a first focal point of the curved mirror to a conjugate second focal point of the curved mirror may be employed in example embodiments as a generalisation of the ellipsoidal mirror 32. The ellipsoidal mirror 32 may be substantially spheroidal in some example embodiments, with some deviation in curvature from the spheroidal form that nevertheless allows the ophthalmic imaging instrument 100 to acquire WF or UWF images of the fundus 12. The rotational axis of the rotatable mirror 30 may, as in the present example embodiment, be parallel to the axis of circular symmetry of the spheroidal mirror. The length of the optical path between the first focal point 32-1, and points along a path T3 on the ellipsoidal mirror 32 followed by the beam of light L_{T} incident thereon as the beam is scanned by rotation of the rotatable mirror 30, is therefore constant. The scanning performed by the scanner 36 and the rotatable mirror 30 may be coordinated by the processor 50 or a scanning system controller (not shown) such that the beam of light L_{T} is scanned across the ocular fundus 12 in accordance with a predefined scan pattern.

The curved mirror 38 (also referred to as a slit mirror) may be an ellipsoidal mirror, as in the present example embodiment. Each of the curved mirror 38 and the ellipsoidal mirror 32 thus has a respective first focal point and a respective conjugate second focal point. The scanner 36 is arranged to reflect the beam of light L_{T} at the first focal point of the curved mirror 38, such that a projection of the beam L_{T} on the curved mirror 38 follows the trajectory T1 shown in Figure 1. The curved mirror 38 reflects the beam of light L_{T} incident thereon to its second focal point, and the rotatable mirror 30 is arranged to reflect the beam of light L_{T} at the second focal point of the curved mirror 38 onto the ellipsoidal mirror 32. The rotatable mirror 30 is also arranged to reflect the beam L_{T} at the first focal point 32-1 of the ellipsoidal mirror 32, and a pupil 14 of the eye 10 is disposed at the second focal point 32-2 of the ellipsoidal mirror 32. As the beam of light L_{T} is scanned across the curved mirror 38, the beam L_{T} is reflected therefrom onto the ellipsoidal mirror 32 via the rotatable mirror 30, such that a projection of the beam L_{T} on the ellipsoidal mirror 32 follows the trajectory T2 shown in Figure 1 during performance of each so-called "vertical scan" along a so-called "vertical" (or "fast") scan direction across the ellipsoidal mirror 32. As the rotatable mirror 30 rotates, it causes the adjacent vertical scans to be displaced from one another along a so-called "horizontal" (or "slow") scan direction, such that points in the vertical scans having the same elevation are arranged along the path T3 illustrated in Figure 1.

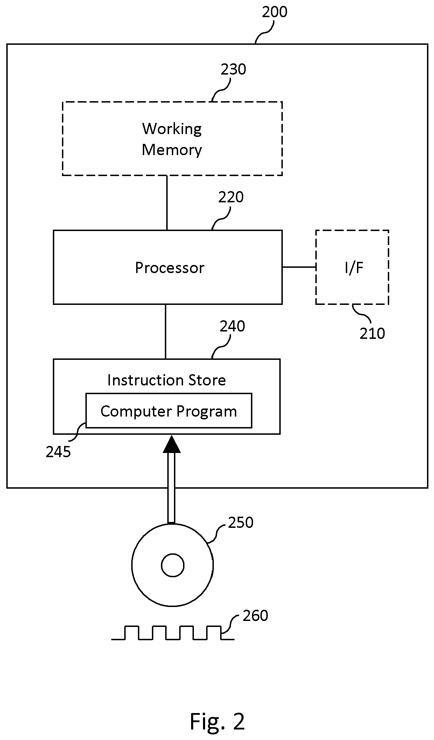

The processor 50 may be provided in any suitable form, for example as a processor 220 of a programmable signal processing hardware 200 of the kind illustrated schematically in Figure 2. The programmable signal processing hardware 200 comprises a communication interface (I/F) 210, for communicating control signals and/or data with the light source 20, the drive mechanism 34, the stereo imaging apparatus 40, the photodetector 70 and a fixation target light source, as described herein. The signal processing hardware 200 further comprises a processor 220 (e.g. a Central Processing Unit, CPU, and/or a Graphics Processing Unit, GPU), a working memory 230 (e.g. a random-access memory) and an instruction store 240 storing a computer program 245 comprising the computer-readable instructions which, when executed by the processor 220, cause the processor 220 to perform various functions of the processor 50 described herein.

The working memory 230 stores information used by the processor 220 during execution of the computer program 245. The instruction store 240 may comprise a ROM (e.g. in the form of an electrically erasable programmable read-only memory (EEPROM) or flash memory) which is pre-loaded with the computer-readable instructions. Alternatively, the instruction store 240 may comprise a RAM or similar type of memory, and the computer-readable instructions of the computer program 245 can be input thereto from a computer program product, such as a non-transitory, computer-readable storage medium 250 in the form of a CD-ROM, DVDROM, etc. or a computer-readable signal 260 carrying the computer-readable instructions. In any case, the computer program 245, when executed by the processor 220, causes the processor 220 to perform the functions of the processor 50 as described herein. More generally, the processor 50 of the present example embodiment may comprise one or more instances of the computer processor 220 and one or more instances of the memory 240 storing computer-readable instructions which, when executed by the computer processor(s) 220, cause the computer processor(s) 220 to perform the functions of the processor 50 as described herein. Where a plurality of processors 220 is provided, the processors 220 may communicate with each other via any kind of computer network.

It should be noted, however, that the processor 50 may alternatively be implemented in non-programmable hardware, such as an ASIC, an FPGA or other integrated circuit dedicated to performing the functions of the processor 50 described herein, or a combination of such non-programmable hardware and programmable hardware as described above with reference to Figure 2.

Referring again to Figure 1, the stereo imaging apparatus 40 may form part of a so-called pupil alignment module (PAM), and comprises a first camera 42 and a second camera 44 that are arranged to acquire respective stereo images 46-1 and 46-2 of the pupil 14 and a surrounding portion of the eye 10 (including at least some of the iris and typically also some of the sclera) via the rotatable mirror 30 and the ellipsoidal mirror 32. The stereo imaging apparatus 40 may, as in the present example embodiment, also have an illumination source 47 which illuminates the eye 10 via the rotatable mirror 30 and the ellipsoidal mirror 32 with light whose reflection from the eye 10 is detected by the first camera 42 and the second camera 44 to acquire the respective stereo images 46-1 and 46-2 of the pupil 14. The illumination is typically in the infra-red (IR) band for patient comfort and to avoid pupil constriction, and may be provided by an illumination source 47 in the form of an IR light-emitting diode (LED), for example. The stereo cameras 42 and 44 may employ complementary metal oxide semiconductor (CMOS) sensors, and fast lenses of fixed focal length to compensate for the sensors' relatively low sensitivity to the IR illumination that is typically provided by the illumination source. The ophthalmic imaging instrument 100 may, as in the present example embodiment, further comprise (e.g. as part of the PAM) a fixation target light source 80 comprising one or more light sources such as light-emitting diodes (LEDs), for example. The fixation target light source 80 is arranged to project fixation light L_{F} onto the fundus 12 via the rotatable mirror 30 and the ellipsoidal mirror 32. A projection of the fixation target light L_{F} on the fundus 12 has a predetermined shape chosen to assist the subject in maintaining a steady gaze direction, which may, for example, include one or more of a crosshair, a dot, a disc or a circle. The colour of the fixation light L_{F} may be variable by the processor 50, as described below.

The processor 50 is arranged to operate in a patient alignment mode to control the drive mechanism 34 to rotate the rotatable mirror 30 to a predetermined orientation (if the rotatable mirror 30 is not already in that orientation), and to hold the rotatable mirror 30 stationary in the predetermined orientation while controlling the stereo imaging apparatus 40 to acquire the stereo images 46-1 and 46-2 of the pupil 14 at the same time (or with a small delay between the acquisitions of the two images that does not significantly affect the stereoscopic distance measurement) via the rotatable mirror 30 and the ellipsoidal mirror 32. The processor 50 controls the stereo imaging apparatus 40 to keep acquiring stereo images of the pupil 14 during operation in the patient alignment mode. The processor 50 is further configured to process the acquired stereo images to generate a signal S for bringing the pupil 14 within a target range suitable for acquiring an UWF image 55 of the fundus 12.

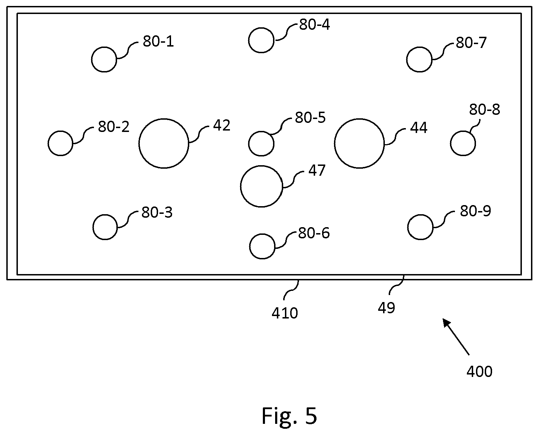

While the rotatable mirror 30 remains stationary in the predetermined orientation in the patient alignment mode (which may also be referred to herein as a fixation mode, or a patient alignment and fixation mode), the processor 50 is also arranged to control the fixation target light source 80 to project the fixation light L_{F} onto the rotatable mirror 30, specifically onto the first focal point 32-1 of the ellipsoidal mirror 32. The fixation target light source 80 is disposed in relation to the rotatable mirror 30 whilst in the predetermined orientation such that the fixation light L_{F} is projected onto the fundus 12 via the rotatable mirror 30 and the ellipsoidal mirror 32 to fix the gaze direction of the eye 10 and thus keep the eye 10 steady while the pupil 14 is brought within the target range suitable for acquiring an ultra-widefield image 55 of the fundus 12 on the basis of the signal S. The fixation target light source 80 may, as in the present example embodiment, be arranged to project the fixation light L_{F} onto the rotatable mirror 30 from a location such that, when the rotatable mirror 30 is in the predetermined orientation, the fixation light L_{F} is incident on the eye 10 in a direction for fixing the gaze direction of the eye 10 in a central gaze direction, which the subject adopts while looking straight ahead. However, as explained below with reference to Figure 5, the fixation target light source 80 may be configured to provide gaze fixation in gaze directions other than the central gaze direction.

In the patient alignment mode, the processor 50 may, as in the present example embodiment, process the stereo images acquired by the stereo imaging apparatus 40 using any known stereoscopic ranging technique which uses the concept of parallax and triangulation to estimate distance, to determine an indication of a distance d between the exit pupil of the ophthalmic imaging instrument 100 and the pupil 14 of the eye 10. For example, the processor 50 may determine the indication of the distance d by processing each image of the acquired stereo images 46-1 and 46-2 to locate a respective pupil centre of the pupil in the image (e.g. using edge detection to determine an outline of the pupil, and fitting a circle to the outline to find the circle centre), mapping the located pupil centres to a common image frame, determining a separation between the pupil centres in the common image frame, and determining the indication of the distance d using the determined separation, which is inversely related to the distance d. The processor 50 may then compare the determined indication of the distance d with predetermined threshold(s) to determine whether the pupil 14 is too close to the exit pupil position of the ophthalmic imaging instrument 100, too far from exit pupil position, or within a predetermined range of distances from the exit pupil position that is acceptable for image acquisition. The processor 50 may, as in the present example embodiment, then generate the signal S to control the fixation target light source 80 to output fixation light of a colour that is indicative of the comparison result. For example, the fixation target light source 80 may be controlled by the processor 50 to emit fixation light of a first colour (e.g. blue) in case the distance between the exit pupil E of the ophthalmic imaging instrument 100 and the pupil 14 is greater than a threshold value demarcating the end of the range R and the pupil 14 is on a side of the range R further from the ophthalmic imaging instrument 100, to emit fixation light of a second, different colour (e.g. red) in case the distance between the exit pupil E and the pupil 14 is greater than the threshold value and the pupil 14 is on the other side of the range R which is nearer to the ophthalmic imaging instrument 100, and to emit fixation light of a yet further colour (e.g. green) in case the distance between the exit pupil E and the pupil 14 is smaller than the threshold value. Although references are made herein to the exit pupil position, any other reference point of the ophthalmic imaging instrument 100, in the vicinity of which the pupil 14 needs to be positioned for the acquisition of the image 55 of the fundus 12 of the eye 10, may be used instead.

Additionally or alternatively, the processor 50 may generate the control signal S to control a speaker (not shown in Figure 1) to generate a sound (e.g. of a varying tone or spoken word(s)) which is indicative of the comparison result. For example, the speaker may be controlled by the processor 50 to generate a first (e.g. low-pitched) tone or a spoken indication, for example, in case the distance between the exit pupil E of the ophthalmic imaging instrument 100 and the pupil 14 is greater than the threshold value and the pupil 14 is on the side of the range R further from the ophthalmic imaging instrument 100, a different, second (e.g. high-pitched) tone or spoken indication in case the distance between the exit pupil E and the pupil 14 is greater than the threshold value and the pupil 14 is on the other side of the range R which is nearer to the ophthalmic imaging instrument 100, and a yet further (e.g. medium-pitched) tone or spoken indication in case the distance between the exit pupil E and the pupil 14 is smaller than the threshold value.

By the processing of each acquired pair of stereo images as described above, the patient may be guided to move the eye 10 backwards (away from the ophthalmic imaging instrument 100) or forwards (towards the ophthalmic imaging instrument 100), as appropriate, until the patient observes the fixation light to be green and/or hears the medium-pitched tone or spoken indication (as the case may be), informing the patient that the pupil 14 of the eye 10 is sufficiently close to the exit pupil (or other reference point) of the ophthalmic imaging instrument 100 for the UWF imaging of the fundus 12 to begin.

After the distance between the exit pupil position and the pupil 14 has been determined to be smaller than the threshold value, the processor 50 is arranged to start operating in a fundus imaging mode to control the ophthalmic imaging instrument 100 to acquire the UWF image 55 of the fundus 12. The processor 50 does this by controlling the light source 20 to start emitting the beam of light L_{T}, and controlling the ophthalmic imaging instrument 100 to acquire the UWF image 55 of the fundus 12 by controlling the scanner 36 and the rotatable mirror 30 to scan the beam of light L_{T} across the fundus 12 via the ellipsoidal mirror 32. The processor 50 preferably controls the fixation target light source 80 to stop emitting the fixation light L_{F} (before or after the light source 20 starts emitting the beam of light L_{T}) to reduce artifacts and otherwise improve the quality of the UWF image 55. To reduce the risk of the eye 10 becoming misaligned with the ophthalmic imaging instrument 100, the processor 50 may, as in the present example embodiment, begin to operate in the fundus imaging mode automatically in response to determining that the distance d between the exit pupil of the ophthalmic imaging instrument 100 and the pupil 14 is smaller than the threshold value. However, in other example embodiments, this switch in the mode of operation may be instructed by an operator of the ophthalmic imaging instrument 100 in reaction to visual, audio and/or tactile feedback based on the signal S, for example by the operator clicking a mouse button or pressing a key on a computer keyboard communicatively coupled to the processor 50.

The stereo imaging apparatus 40 may, as in the present example embodiment, be located within a region G between the rotatable mirror 30 and the ellipsoidal mirror 32 which is not traversed by the beam of light L_{T} during acquisition of the UWF image 55 of the fundus 12. The fixation target light source 80 may, as in the present example embodiment, also be located within the region R. The stereo imaging apparatus 40 and fixation target light source 80 may thus be accommodated in a region within the bowl of the ellipsoidal mirror 32 which is not used for beam propagation. This arrangement may advantageously allow the ophthalmic imaging instrument 100 to be made more compact, by placing the stereo imaging apparatus 40 and the fixation target light source 80 in an otherwise unused region of the ophthalmic imaging instrument 100.

Furthermore, accommodating the stereo imaging apparatus 40 and the fixation target light source 80 in the region G allows for the option of orientating the rotatable mirror 30 in the patient alignment mode (i.e. choosing the predetermined orientation mentioned above) such that the rotatable mirror 30 starts rotating from the predetermined orientation as soon as the UWF ophthalmic imaging instrument 100 starts acquiring the UWF image 55 during operation of the processor 50 in the fundus imaging mode. A delay in the start of image acquisition, which would be caused by a rotation of the rotatable mirror 30 from the predetermined orientation used in the patient alignment mode to a (different) start orientation for image acquisition, may therefore be avoided, and the risk of the gaze direction of the eye 10 changing thus reduced. However, if this advantage is outweighed by the need to provide easy access to the stereo imaging apparatus 40 or the fixation target light source 80 for maintenance and/or adjustment, for example, the stereo imaging apparatus 40 and the fixation target light source 80 may instead be located outside the bowl of the ellipsoidal mirror 32 in some example embodiments, with the predetermined orientation of the rotatable mirror 30 used in the patient alignment mode being set to still allow the stereo imaging apparatus 40 to acquire the stereo images 46-1 and 46-2 of the pupil 14 via the rotatable mirror 30 and the ellipsoidal mirror 32 while the eye 10 remains fixated. For example, the stereo imaging apparatus 40 and the fixation target light source 80 may be located above the rim of the ellipsoidal mirror (similarly to the curved mirror 38 in Figure 1, for example) so as to each have a line of sight to the rotatable mirror 30. Alternatively, the stereo imaging apparatus 40 and the fixation target light source 80 may be located on the non-reflecting side of the ellipsoidal mirror 32, with one or more holes being provided in the ellipsoidal mirror 32 to allow light to pass through the ellipsoidal mirror 32 to the stereo imaging apparatus 40 and from the fixation target light source 80.

Regardless of whether the rotatable mirror 30 is orientated in the patient alignment mode to allow the rotatable mirror 30 to start rotating from the predetermined orientation when the UWF ophthalmic imaging instrument 100 starts acquiring the UWF image 55, it may be advantageous to locate the stereo imaging apparatus 40 between the rotatable mirror 30 and the ellipsoidal mirror 32 at a location which minimises a deviation from circularity of respective representations of the pupil 14 in the stereo images 46-1 and 46-2 of the pupil 14 acquired by the stereo imaging apparatus 40 when the rotatable mirror 30 is in the predetermined orientation. Such a location of the stereo imaging apparatus 40 to bring its viewpoint to be as close as possible to a central gaze direction of the eye 10, and thus allow the pupil 14 in the images 46-1 and 46-2 to be as circular as possible, may facilitate the process of locating the pupil centre and therefore an accurate calculation of the distance between the pupil 14 and the exit pupil of the ophthalmic imaging instrument 100.

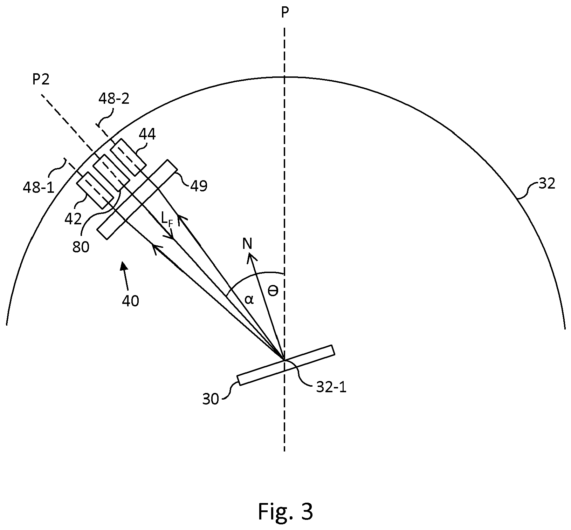

By way of an example, the ellipsoidal mirror 32 of the present example embodiment has a plane of symmetry P comprising the first focal point 32-1 and the second focal point 32-2, and a normal direction N of the rotatable mirror 30 at the first focal point 32-1 subtends a predetermined angle θ relative to the plane of symmetry P when the rotatable mirror 30 is in the predetermined orientation, as illustrated in Figure 3. For clarity, the sizes of angle θ and angle α discussed below have been exaggerated in Figure 3. The first camera 42 and the second camera 44 are arranged symmetrically about a second plane P2, which passes through the first focal point 32-1 and the second focal point 32-2. The first camera 42 and the second camera 44 may, as in the present example embodiment, comprise respective camera lenses having respective optical axes 48-1 and 48-2 that are parallel to each other and arranged equidistantly from, and on opposite sides of, the second plane P2. However, in other example embodiments, the optical axes 48-1 and 48-2 may both be within the second plane P2. Where the normal direction N of the rotatable mirror 30 and the second plane P2 subtend an angle α at the first focal point 32-1 which is less than two times as large as the predetermined angle θ, the deviation from circularity of respective representations of the pupil 14 in the stereo images 46-1 and 46-2 of the pupil 14 acquired using this arrangement was found to be small enough to allow satisfactory pupil alignment to be achieved.

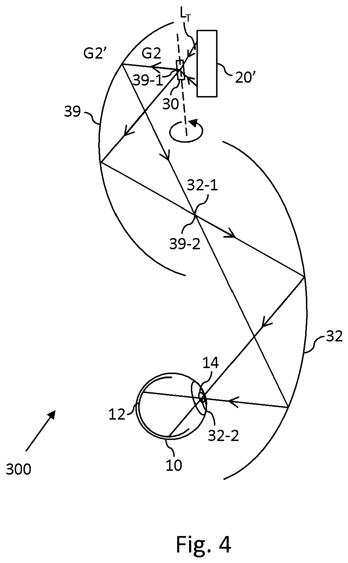

It should be noted that, although the rotatable mirror 30 is provided to reflect the beam of light L_{T} across the ellipsoidal mirror 32 at the first focal point 32-1 of the ellipsoidal mirror 32, the optical system of the ophthalmic imaging instrument 100 may be arranged such that the rotatable mirror 30 scans the beam L_{T} via the first focal point 32-1 in another way. Figure 4 is a schematic illustration of a line-scan UWF ophthalmic imaging instrument 300 according to a variant of the example embodiment which comprises, in addition to the ellipsoidal mirror 32, a second ellipsoidal mirror 39, via which the rotatable mirror 30 is arranged to scan a beam of light L_{T}' in the form of a line of light across the fundus 12. In this case, the beam of light L_{T}' has a projection onto a plane normal to its direction of propagation (i.e. a cross-section) which is a line, and may be generated from a beam of light having a cross-section of a spot (as generated by the light source 20) using a cylindrical lens, or may be generated using another optical arrangement for generating line-field illumination known to those versed in the art (e.g. a back-lit slit aperture). As illustrated in Figure 4, the second ellipsoidal mirror 39 has a first focal point 39-1 and a second focal point 39-2, with the rotatable mirror 30 being arranged to reflect the beam of light L_{T}' at the first focal point 39-1 of the second ellipsoidal mirror 39. The second focal point 39-2 of the second ellipsoidal mirror 39 coincides with the first focal point 32-1 of the ellipsoidal mirror 32.

The light source 20', which generates the line-field illumination L_{T}', is shown in Figure 4, and this is provided instead of the light source 20, the scanner 36 and the curved mirror 38 of the example embodiment of Figure 1. The line-scan UWF ophthalmic imaging instrument 300 includes the remaining components of the UWF ophthalmic imaging instrument 100, although these are not shown in Figure 4 to more clearly illustrate the different scan transfer arrangement comprising the two ellipsoidal mirrors 32 and 39. The line-scan UWF ophthalmic imaging instrument 300 also has a beam splitter to direct the return light from the fundus 12 to a photodetector for detecting the return line-field illumination, although this has similarly been omitted from Figure 4 for clarity.

In the line-scan UWF ophthalmic imaging instrument 300, the stereo imaging apparatus 40 and the fixation target light source 80 (where provided) may be accommodated in a region G2 (between the second ellipsoidal mirror 39 and the rotatable mirror 30) not traversed by the beam of light L_{T}'. The stereo imaging apparatus 40 and the fixation target light source 80 (where provided) may be arranged in relation to the rotatable mirror 30 and configured to operate as described above, although with the light propagating to the stereo imaging apparatus 40 from the eye 10, and the fixation light L_{F} propagating from the fixation target light source 80 to the eye 10, doing so via the second ellipsoidal mirror 39 as well as the ellipsoidal mirror 32.

Alternatively, if there is insufficient space within the bowl of the second ellipsoidal mirror 39, one or both of the stereo imaging apparatus 40 and the fixation target light source 80 may be located in a region G2' on the non-reflecting side of the second ellipsoidal mirror 39, with one or more holes being provided in the second ellipsoidal mirror 39 to allow light to pass through the second ellipsoidal mirror 39 to the stereo imaging apparatus 40 and from the fixation target light source 80.

Regardless of whether the stereo imaging apparatus 40 is accommodated within the region G (or region G2 in the variant of Figure 4), the stereo imaging apparatus 40 may, as in the present example embodiment, further comprise a Fresnel lens 49 disposed between the rotatable mirror 30 and both the first camera 42 and the second camera 44. In the example embodiment of Figure 1, the Fresnel lens 49 is arranged to refract light from the eye 10, which has been reflected by the rotatable mirror 30 at the first focal point 32-1 of the ellipsoidal mirror 32, to propagate along a first optical axis 48-1 of the first camera 42, and along a second optical axis 48-2 of the second camera 44. The Fresnel lens 49 allows the first camera 42 and the second camera 44 to be easily mounted on a supporting circuit board or other flat substrate, with their respective optical axes being normal to the surface of the substrate. The time-consuming process of carefully aligning the optical axes of the first camera 42 and the second camera 44 to pass through the first focal point 32-1 of the ellipsoidal mirror 32, which may otherwise need to be performed to improve the stereoscopic ranging, may be avoided, and the manufacture of the stereo imaging apparatus 40 consequently made simpler and faster. Similarly, in the variant described above with reference to Figure 4, the Fresnel lens 49 may be disposed between the rotatable mirror 30 and both the first camera 42 and the second camera 44 that are located in the region G2, and arranged to refract light from the eye 10, which has been reflected by the rotatable mirror 30 at the first focal point 39-1 of the second ellipsoidal mirror 39, to propagate along a first optical axis 48-1 of the first camera 42, and along a second optical axis 48-2 of the second camera 44.

Although the fixation target light source 80 is operable to project the fixation light L_{F} onto the rotatable mirror 30 from a single location for central gaze fixation in the example embodiment and the variant thereof described above, the fixation target light source 80 may alternatively be configured to project the fixation light L_{F} onto the rotatable mirror 30 from a selected location of a plurality of locations on the fixation target light source 80 that are arranged in different respective directions from the first focal point 32-1 of the ellipsoidal mirror 32 (or from the first focal point 39-1 of the second ellipsoidal mirror 39 in the variant of Figure 4).

Figure 5 is a schematic illustration of a PAM 400 comprising the stereo imaging system 40 and a modified version of the fixation target light source 80 included in the example embodiment of Figure 1, when viewed from the rotatable mirror 30. As shown in Figure 5, the PAM 400 comprises a flat substrate 410, onto which are mounted the first camera 42 and the second camera 44, as well as an IR illumination source 47 for illuminating the eye 10 so that it can be imaged by the stereo cameras 42 and 44. The fixation target light source 80 comprises a plurality of separate fixation light sources in the form of LEDs, labelled 80-1 to 80-9 in Figure 5. Each of these fixation target light sources may generate visible light of at least three colours, for example red, green and blue. In the example of Figure 5, the LEDs 80-1 to 80-9 are RGB LEDs, each capable of emitting red, green or blue light under the control of the processor 50. Although there are nine such light sources which are arranged as shown in the example of Figure 5, neither their number not arrangement is so limited. The PAM 400 may, as in the present example, also include the Fresnel lens 49 mentioned above, which is overlaid on the stereo cameras 42 and 44, the IR illumination source 47 and the LEDs 80-1 to 80-9 so that it is disposed between these components and the rotatable mirror 30.

The LEDs 80-1 to 80-9 are provided at different respective locations that are arranged in different respective directions from the first focal point 32-1 in the example embodiment of Figure 1 (or the first focal point 39-1 of the second ellipsoidal mirror 39 in the variant of Figure 4) so that the eye 10 can be steered in different directions by fixating on the light that is relayed to the eye 10 via the rotatable mirror 30 and the ellipsoidal mirror 32 (and the second ellipsoidal mirror 39 in the variant of Figure 4) from a selected one of the LEDs. In the example of Figure 5, LED 80-5 is arranged to provide central gaze fixation, while the remaining LEDs are arranged to guide the patient to look left, right, straight up, up and to the left, up and to the right, straight down, down and to the left, and down and to the right.

It should be noted, however, that the fixation target light source 80 may be arranged in other ways to project the fixation light L_{F} onto the rotatable mirror 30 from a selected location of a plurality of locations on the fixation target light source 80 that are arranged in different respective directions from the first focal point 32-1 of the ellipsoidal mirror 32 (or from the first focal point 39-1 of the second ellipsoidal mirror 39 in the variant of Figure 4). For example, the fixation target light source 80 may be provided in the alternative form of at least one display screen (e.g. a liquid crystal display (LCD), an LED display screen of an organic LED (OLED) display screen) which is controllable by the processor 50 to project the fixation light L_{F} from a selected portion of a plurality of separated portions on the display screen, and in some cases in a selected colour of a plurality of available colours. As another example, the fixation target light source 80 may be provided in the form of an arrangement of optical fibres, which may emerge from the substrate 410 at the same locations as the locations of the LEDs 80-1 to 80-9 and project light being guided therethrough towards the rotatable mirror 30, wherein optical switches or other means controllable by the processor 50 are provided to allow fixation light L_{F} from a selected optical fibre to be incident on the rotatable mirror 30.

In the patient alignment mode, the processor 50 is arranged to select a gaze direction from a plurality of different gaze directions, in which gaze direction a gaze direction of the eye 10 is to be fixed, and determine, using the selected gaze direction, a corresponding location of the plurality of locations, from which location the fixation target light source 80 is to project the fixation light L_{F} onto the rotatable mirror 30. In the example of Figure 5, the processor 50 may selected one of the LEDs 80-1 to 80-9, which corresponds to the selected gaze direction. The processor 50 may then control the fixation target light source 80 to project the fixation light L_{F} onto the rotatable mirror 30 from the determined location to fix the gaze direction of the eye 10 in the selected gaze direction, for example by driving only the selected LED to emit the target fixation light L_{F}.

The plurality of different gaze directions may comprise a central gaze direction, and the plurality of locations on the fixation target light source 80 may comprises a central location (i.e. the location of LED 80-5 in the example of Figure 5) for fixing the gaze direction of the eye 10 in the central gaze direction when the fixation light L_{F} is projected onto the fundus 12 from the central location via the rotatable mirror 30 in the predetermined orientation and via the ellipsoidal mirror 32. For each location of the locations on the fixation target light source 80 other than the central location, the Fresnel lens 49 is arranged to refract the fixation light L_{F}, when emitted from the location, to propagate towards a common point on the rotatable mirror 30. This common point corresponds to the first focal point of the ellipsoidal mirror 32 in the above example embodiment, and the first focal point 39-1 of the second ellipsoidal mirror 39 in the variant thereof described with reference to Figure 4. Accordingly, the Fresnel lens 49 can provide the necessary focussing of both the return light from the eye 10 used to form the stereo images 46-1 and 46-2 and the fixation light L_{F} used to fix the gaze direction of the eye 10, thereby avoiding the need to provide separate optics for the stereo imaging apparatus 40 and the fixation target light source 80.

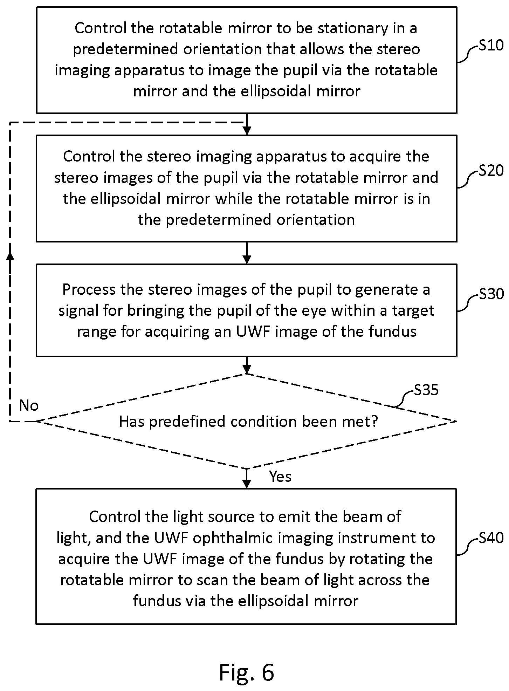

Figure 6 is a flow diagram summarising the operations performed by the processor 50 as described above to control the ophthalmic imaging instrument 100 to acquire an UWF image 55 of the fundus 12. For clarity, some actions and processes that may be taken/performed in the context of these operations as described above will not be described again here but are understood to form optional features of the operations described in the following with reference to Figure 6. The processor 50 may similarly control the ophthalmic imaging instrument 300 to acquire an UWF image 55 of the fundus 12.

The processor 50 operates in the patient alignment mode to generate a signal S to bring the pupil 14 of the eye 10 within a target range for acquiring the UWF image 55 of the fundus 12 by firstly controlling (in S10 of Figure 6) the rotatable mirror 30 to be stationary in a predetermined orientation that allows the stereo imaging apparatus 40 to image the pupil 14 via the rotatable mirror 30 and the ellipsoidal mirror 32. The processer 50 may then control the illumination source of the stereo imaging apparatus 40 to turn on, thereby illuminating the eye 10 with IR light via the rotatable mirror 30 and the ellipsoidal mirror 32 (and via the second ellipsoidal mirror 39 in the variant of Figure 4).

Then, in S20 of Figure 6, the processor 50 controls the stereo imaging apparatus 40 to acquire the stereo images 46-1 and 46-2 of the pupil 14 via the rotatable mirror 30 and the ellipsoidal mirror 32 while the rotatable mirror 30 is in the predetermined orientation. In the context of the variant of Figure 4, the processor 50 would control the stereo imaging apparatus 40 in S20 to acquire the stereo images 46-1 and 46-2 of the pupil 14 via the rotatable mirror 30, the second ellipsoidal mirror 39 and the ellipsoidal mirror 32 while the rotatable mirror 30 is in the predetermined orientation. Whilst the rotatable mirror 30 is in the predetermined orientation and the stereo imaging apparatus 40 is being controlled to acquire the stereo images 46-1 and 46-2 of the pupil 14, the processor 50 may (as an optional part of process S20 of Figure 6) control the fixation target light source 80 to project the fixation light L_{F} onto the rotatable mirror 30, the fixation target light source 80 being disposed in relation to the rotatable mirror 30 such that the fixation light L_{F} is projected onto the fundus 12 via the ellipsoidal mirror 32 to fix a gaze direction of the eye 10.

In S30 of Figure 6, the processor processes the stereo images 46-1 and 46-2 of the pupil 14 as described above to generate the signal S.

In an optional process S35, the processor 50 may determine whether a predefined condition has been met. The predefined condition may, for example, be that the processor 50 has received an instruction provided by the operator of the ophthalmic imaging instrument 100, who is being provided with visual and/or audio feedback based on the signal S, to start imaging the fundus 12. As another example, the predefined condition may be that the distance between the exit pupil of the ophthalmic imaging instrument 100 and the pupil 14 (as determined by the processor 50 based on the stereo images 46-1 and 46-2) is within the target range suitable for acquiring an UWF image 55 of the fundus 12. As a further alternative, the predefined condition may be that a predetermined time has elapsed from the start of operation of the processor 50 in the patient alignment mode after which the distance between the exit pupil of the ophthalmic imaging instrument 100 and the pupil 14 is likely to be within the target range. If the processor determines that the predefined condition has not been met, the process loops back to S20, otherwise it proceeds to S40 in Figure 6.

In S40 of Figure 6, the processor 50 controls the light source 20 to emit the beam of light L_{T}, and controls the ophthalmic imaging instrument 100 to acquire the UWF image 55 of the fundus 12 by rotating the rotatable mirror 30 to scan the beam of light L_{T} across the fundus 12 via the ellipsoidal mirror 32 (and via the second ellipsoidal mirror 39 in the variant of Figure 4). Prior to the acquisition of the UWF image 55, the processor 50 preferably turns off the fixation target light source 80 and the IR illumination source 47.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the functions of the processor 50, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example of the invention, as described above.

Included in the programming and/or software of the system are software modules for implementing the procedures described herein. In some example embodiments herein, a module includes software, although in other example embodiments herein, a module includes hardware, or a combination of hardware and software.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable subcombination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. An ultra-widefield ophthalmic imaging instrument (100; 300) arranged to acquire an ultra-widefield image (55) of a fundus (12) of an eye (10) by scanning a beam of light (L_{T}) across the fundus (12), comprising:
a light source (20; 20') arranged to emit the beam of light (L_{T}; L_{T}');
a rotatable mirror (30) arranged to scan the beam of light (L_{T}; L_{T}') across the fundus (12);
an ellipsoidal mirror (32) via which the rotatable mirror (30) is arranged to scan the beam of light (L_{T}; L_{T}') across the fundus (12), the ellipsoidal mirror (32) having a first focal point (32-1) and a second focal point (32-2), wherein the rotatable mirror (30) is arranged to scan the beam of light (L_{T}; L_{T}') across the ellipsoidal mirror (32) via the first focal point (32-1), and a pupil (14) of the eye (10) is positioned at the second focal point (32-2) during use of the ultra-widefield ophthalmic imaging instrument (100);
a stereo imaging apparatus (40) arranged to acquire stereo images (46-1; 46-2) of the pupil (14) via the rotatable mirror (30) and the ellipsoidal mirror (32); and
a processor (50) arranged to operate in a patient alignment mode to generate, based on the stereo images (46-1; 46-2) of the pupil (14), a signal (S) for bringing the pupil (14) of the eye (10) within a target range for acquiring an ultra-widefield image (55) of the fundus (12), and to subsequently operate in a fundus imaging mode to control the ultra-widefield ophthalmic imaging instrument (100) to acquire the ultra-widefield image (55) of the fundus (12), wherein
in the patient alignment mode, the processor (50) is arranged to:
control the rotatable mirror (30) to be stationary in a predetermined orientation;
control the stereo imaging apparatus (40) to acquire the stereo images (46-1; 46-2) of the pupil (14) via the rotatable mirror (30) and the ellipsoidal mirror (32) while the rotatable mirror (30) is stationary in the predetermined orientation; and
process the stereo images (46-1; 46-2) of the pupil (14) to generate the signal (S) for bringing the pupil (14) of the eye (10) into alignment with the imaging position; and
in the fundus imaging mode, the processor (50) is arranged to:
control the light source (20; 20') to emit the beam of light (L_{T}; L_{T}'); and
control the ultra-widefield ophthalmic imaging instrument (100; 300) to acquire the ultra-widefield image (55) of the fundus (12) by controlling the rotatable mirror (30) to scan the beam of light (L_{T}; L_{T}') across the fundus (12) via the ellipsoidal mirror (32).

2. The ultra-widefield ophthalmic imaging instrument (100) according to Claim 1, wherein the rotatable mirror (30) is arranged to reflect the beam of light (L_{T}) at the first focal point (32-1), and the stereo imaging apparatus (40) is located within a region (R) between the rotatable mirror (30) and the ellipsoidal mirror (32) not traversed by the beam of light (L_{T}) during acquisition of the ultra-widefield image (55) of the fundus (12).

3. The ultra-widefield ophthalmic imaging instrument (100) according to Claim 2, wherein the predetermined orientation of the rotatable mirror (30) is such that the rotatable mirror (30) starts rotating from the predetermined orientation when the ultra-widefield ophthalmic imaging instrument (100) starts acquiring the ultra-widefield image (55) of the fundus (12).

4. The ultra-widefield ophthalmic imaging instrument (100) according to Claim 2 or Claim 3, wherein the stereo imaging apparatus (40) is located between the rotatable mirror (30) and the ellipsoidal mirror (32) at a location which minimises a deviation from circularity of respective representations of the pupil (14) in the stereo images (46-1; 46-2) of the pupil (14) acquired by the stereo imaging apparatus (40) when the rotatable mirror (30) is in the predetermined orientation.

5. The ultra-widefield ophthalmic imaging instrument (100) according to Claim 4, wherein
the ellipsoidal mirror (32) has a plane of symmetry (P) comprising the first focal point (32-1) and the second focal point (32-2),
a normal direction of the rotatable mirror (30) at the first focal point (32-1) subtends a predetermined angle relative to the plane of symmetry (P) when the rotatable mirror (30) is in the predetermined orientation, and
the stereo imaging apparatus (40) comprises a first camera (42) and a second camera (44), wherein the first camera (42) and the second camera (44) are arranged equidistantly from and on opposite sides of a second plane (P2) comprising the first focal point (32-1) and the second focal point (32-2), wherein the normal direction of the rotatable mirror (30) and the second plane (P2) subtend an angle at the first focal point (32-1) which is less than two times as large as the predetermined angle.

6. The ultra-widefield ophthalmic imaging instrument (100) according to Claim 5, wherein the first camera (42) has a first optical axis (48-1), and the second camera (44) has a second optical axis (48-2) which is parallel to the first optical axis (48-1), and the stereo imaging apparatus (40) further comprises a Fresnel lens (49) disposed between the rotatable mirror (30) and both the first camera (42) and the second camera (44), the Fresnel lens (49) being arranged to refract light from the eye (10), which has been reflected by the rotatable mirror (30) at the first focal point (32-1) of the ellipsoidal mirror (32), to propagate along the first optical axis (48-1) and the second optical axis (48-2).

7. The ultra-widefield ophthalmic imaging instrument (100) according to any of Claims 1 to 4, wherein the stereo imaging apparatus (40) comprises:
a first camera (42) having a first optical axis (48-1), and a second camera (44) having a second optical axis (48-2) which is parallel to the first optical axis (48-1); and
a Fresnel lens (49) disposed between the rotatable mirror (30) and both the first camera (42) and the second camera (44), the Fresnel lens (49) being arranged to refract light from the eye (10), which has been reflected by the rotatable mirror (30), to propagate along the first optical axis (48-1) and the second optical axis (48-2).

8. The ultra-widefield ophthalmic imaging instrument (100) according to any preceding claim, further comprising:
a fixation target light source (80) arranged to project fixation light (L_{F}) onto the fundus (20) via the rotatable mirror (30) and the ellipsoidal mirror (32),
wherein, in the patient alignment mode, the processor (50) is arranged to control the fixation target light source (80) to project the fixation light (L_{F}) onto the rotatable mirror (30) in the predetermined orientation, the fixation target light source (80) being disposed in relation to the rotatable mirror (30) such that the fixation light (L_{F}) is projected onto the fundus (12) via the ellipsoidal mirror (32) to fix a gaze direction of the eye (10).

9. The ultra-widefield ophthalmic imaging instrument (100) according to Claim 8 when dependent on Claim 2, wherein the fixation target light source (80) is located within the region (R) between the rotatable mirror (30) and the ellipsoidal mirror (32) not traversed by the beam of light (L_{T}) during acquisition of the ultra-widefield image (55) of the fundus (12).

10. The ultra-widefield ophthalmic imaging instrument (100) according to Claim 9, wherein the predetermined orientation is such that the rotatable mirror (30) starts rotating from the predetermined orientation when the ultra-widefield ophthalmic imaging instrument (100) starts acquiring the ultra-widefield image (55) of the fundus (12).

11. The ultra-widefield ophthalmic imaging instrument (100) according to Claim 10, wherein the fixation target light source (80) is arranged to project the fixation light (L_{F}) onto the rotatable mirror (30) from a location such that, when the rotatable mirror (30) is in the predetermined orientation, the fixation light (L_{F}) is incident on the eye (10) in a direction for central gaze fixation of the eye (10).

12. The ultra-widefield ophthalmic imaging instrument (100) according to any preceding claim, further comprising an illumination source (47) arranged to illuminate the eye (10) via the rotatable mirror (30) and the ellipsoidal mirror (32) with light for acquiring the stereo images (46-1; 46-2) of the pupil (14) by the stereo imaging apparatus (40).

13. The ultra-widefield ophthalmic imaging instrument (100) according to any preceding claim, wherein the ultra-widefield ophthalmic imaging instrument (100) comprises an ultra-widefield scanning laser ophthalmoscope.

14. A method of controlling an ultra-widefield ophthalmic imaging instrument (100; 300) to acquire an ultra-widefield image (55) of a fundus (12) of an eye (10) by scanning a beam of light (L_{T}; L_{T}') across the fundus (12), the ultra-widefield ophthalmic imaging instrument (100) comprising:
a light source (20; 20') arranged to emit the beam of light (L_{T}; L_{T}');
a rotatable mirror (30) arranged to scan the beam of light (L_{T}; L_{T}') across the fundus (12);
an ellipsoidal mirror (32) via which the rotatable mirror (30) is arranged to scan the beam of light (L_{T}; L_{T}') across the fundus (12), the ellipsoidal mirror (32) having a first focal point (32-1) and a second focal point (32-2), wherein the rotatable mirror (30) is arranged to scan the beam of light (L_{T}; L_{T}') across the ellipsoidal mirror (32) via the first focal point (32-1) and a pupil (14) of the eye (10) is positioned at the second focal point (32-2) during use of the ultra-widefield ophthalmic imaging instrument (100); and
a stereo imaging apparatus (40) arranged to acquire stereo images (46-1; 46-2) of the pupil (14) via the rotatable mirror (30) and the ellipsoidal mirror (32),
the method comprising:
generating a signal (S) to bring the pupil (14) of the eye (10) within a target range for acquiring the ultra-widefield image (55) of the fundus (12), by:
controlling (S10) the rotatable mirror (30) to be stationary in a predetermined orientation that allows the stereo imaging apparatus (40) to image the pupil (14) via the rotatable mirror (30) and the ellipsoidal mirror (32);
controlling (S20) the stereo imaging apparatus (40) to acquire the stereo images (46-1; 46-2) of the pupil (14) via the rotatable mirror (30) and the ellipsoidal mirror (32) while the rotatable mirror (30) is in the predetermined orientation; and
processing (S30) the stereo images (46-1; 46-2) of the pupil (14) to generate the signal (S); and
after generating the signal (S), controlling (S40) the light source (20; 20') to emit the beam of light (L_{T}; L_{T}'), and controlling the ultra-widefield ophthalmic imaging instrument (100) to acquire the ultra-widefield image (55) of the fundus (12) by rotating the rotatable mirror (30) to scan the beam of light (L_{T}; L_{T}') across the fundus (12) via the ellipsoidal mirror (32).

15. The method according to Claim 14, wherein
the ultra-widefield ophthalmic imaging instrument (100) further comprises a fixation target light source (80) arranged to project fixation light (L_{F}) onto the fundus (20) via the rotatable mirror (30) and the ellipsoidal mirror (32), and
the method further comprises:
whilst the rotatable mirror (30) is in the predetermined orientation and the stereo imaging apparatus (40) is being controlled to acquire the stereo images (46-1; 46-2) of the pupil (14), controlling the fixation target light source (80) to project the fixation light (L_{F}) onto the rotatable mirror (30), the fixation target light source (80) being disposed in relation to the rotatable mirror (30) such that the fixation light (L_{F}) is projected onto the fundus (12) via the ellipsoidal mirror (32) to fix a gaze direction of the eye (10).

## Patentansprüche

1. Ophthalmisches Ultraweitfeld-Bildgebungsinstrument (100; 300), eingerichtet zum Erfassen eines Ultraweitfeld-Bildes (55) eines Fundus (12) eines Auges (10) durch Abtasten eines Lichtstrahls (LT) über den Fundus (12), umfassend:
eine Lichtquelle (20; 20'), eingerichtet zum Emittieren des Lichtstrahls (LT; LT');
einen drehbaren Spiegel (30), eingerichtet zum Abtasten des Lichtstrahls (LT; LT') über den Fundus (12);
einen ellipsoidalen Spiegel (32), mittels dessen der drehbare Spiegel (30) zum Abtasten des Lichtstrahls (LT; LT') über den Fundus (12) eingerichtet ist, wobei der ellipsoidale Spiegel (32) einen ersten Brennpunkt (32-1) und einen zweiten Brennpunkt (32-2) aufweist, wobei der drehbare Spiegel (30) zum Abtasten des Lichtstrahls (LT; LT') über den ellipsoidalen Spiegel (32) durch den ersten Brennpunkt (32-1) eingerichtet ist und eine Pupille (14) des Auges (10) während der Verwendung des ophthalmischen Ultraweitfeld-Bildgebungsinstruments (100) am zweiten Brennpunkt (32-2) positioniert ist;
eine Stereobildgebungsvorrichtung (40), eingerichtet zum Erfassen von Stereobildern (46-1; 46-2) der Pupille (14) mittels des drehbaren Spiegels (30) und des ellipsoidalen Spiegels (32); und
einen Prozessor (50), eingerichtet zum Betrieb in einem Patientenausrichtungsmodus, um basierend auf den Stereobildern (46-1; 46-2) der Pupille (14) ein Signal (S) zum Bringen der Pupille (14) des Auges (10) in einen Zielbereich zum Erfassen eines Ultraweitfeld-Bildes (55) des Fundus (12) zu erzeugen, und zum anschließenden Betrieb in einem Fundusbildgebungsmodus, um das ophthalmische Ultraweitfeld-Bildgebungsinstrument (100) zum Erfassen des Ultraweitfeld-Bildes (55) des Fundus (12) zu steuern, wobei
im Patientenausrichtungsmodus der Prozessor (50) eingerichtet ist zum:
Steuern des drehbaren Spiegels (30), um feststehend in einer vorbestimmten Orientierung zu sein;
Steuern der Stereobildgebungsvorrichtung (40) zum Erfassen der Stereobilder (46-1; 46-2) der Pupille (14) mittels des drehbaren Spiegels (30) und des ellipsoidalen Spiegels (32), während der drehbare Spiegel (30) feststehend in der vorbestimmten Orientierung ist; und
Verarbeiten der Stereobilder (46-1; 46-2) der Pupille (14), um das Signal (S) zum Bringen der Pupille (14) des Auges (10) in Ausrichtung mit der Bildgebungsposition zu erzeugen; und
im Fundusbildgebungsmodus der Prozessor (50) eingerichtet ist zum:
Steuern der Lichtquelle (20; 20') zum Emittieren des Lichtstrahls (LT; LT'); und
Steuern des ophthalmischen Ultraweitfeld-Bildgebungsinstruments (100; 300) zum Erfassen des Ultraweitfeld-Bildes (55) des Fundus (12) durch Steuern des drehbaren Spiegels (30) zum Abtasten des Lichtstrahls (LT; LT') über den Fundus (12) mittels des ellipsoidalen Spiegels (32).

2. Ophthalmisches Ultraweitfeld-Bildgebungsinstrument (100) nach Anspruch 1, wobei der drehbare Spiegel (30) eingerichtet zum Reflektieren des Lichtstrahls (LT) am ersten Brennpunkt (32-1) ist und die Stereobildgebungsvorrichtung (40) innerhalb eines Bereichs (R) zwischen dem drehbaren Spiegel (30) und dem ellipsoidalen Spiegel (32) angeordnet ist, der während der Erfassung des Ultraweitfeld-Bildes (55) des Fundus (12) nicht vom Lichtstrahl (LT) durchquert wird.

3. Ophthalmisches Ultraweitfeld-Bildgebungsinstrument (100) nach Anspruch 2, wobei die vorbestimmte Orientierung des drehbaren Spiegels (30) derart ist, dass der drehbare Spiegel (30) beginnt, sich aus der vorbestimmten Orientierung zu drehen, wenn das ophthalmische Ultraweitfeld-Bildgebungsinstrument (100) beginnt, das Ultraweitfeld-Bild (55) des Fundus (12) zu erfassen.

4. Ophthalmisches Ultraweitfeld-Bildgebungsinstrument (100) nach Anspruch 2 oder Anspruch 3, wobei die Stereobildgebungsvorrichtung (40) zwischen dem drehbaren Spiegel (30) und dem ellipsoidalen Spiegel (32) an einer Position angeordnet ist, die eine Abweichung von der Kreisförmigkeit jeweiliger Darstellungen der Pupille (14) in den Stereobildern (46-1; 46-2) der Pupille (14), die von der Stereobildgebungsvorrichtung (40) erfasst werden, wenn der drehbare Spiegel (30) in der vorbestimmten Orientierung ist, minimiert.

5. Ophthalmisches Ultraweitfeld-Bildgebungsinstrument (100) nach Anspruch 4, wobei
der ellipsoidale Spiegel (32) eine Symmetrieebene (P) aufweist, die den ersten Brennpunkt (32-1) und den zweiten Brennpunkt (32-2) umfasst,
eine Normalenrichtung des drehbaren Spiegels (30) am ersten Brennpunkt (32-1) einen vorbestimmten Winkel relativ zur Symmetrieebene (P) einschließt, wenn der drehbare Spiegel (30) in der vorbestimmten Orientierung ist, und
die Stereobildgebungsvorrichtung (40) eine erste Kamera (42) und eine zweite Kamera (44) umfasst, wobei die erste Kamera (42) und die zweite Kamera (44) äquidistant von und auf gegenüberliegenden Seiten einer zweiten Ebene (P2) angeordnet sind, die den ersten Brennpunkt (32-1) und den zweiten Brennpunkt (32-2) umfasst, wobei die Normalenrichtung des drehbaren Spiegels (30) und die zweite Ebene (P2) am ersten Brennpunkt (32-1) einen Winkel einschließen, der weniger als doppelt so groß wie der vorbestimmte Winkel ist.

6. Ophthalmisches Ultraweitfeld-Bildgebungsinstrument (100) nach Anspruch 5, wobei die erste Kamera (42) eine erste optische Achse (48-1) aufweist und die zweite Kamera (44) eine zweite optische Achse (48-2) aufweist, die parallel zur ersten optischen Achse (48-1) ist, und die Stereobildgebungsvorrichtung (40) ferner eine Fresnel-Linse (49) umfasst, die zwischen dem drehbaren Spiegel (30) und sowohl der ersten Kamera (42) als auch der zweiten Kamera (44) angeordnet ist, wobei die Fresnel-Linse (49) eingerichtet zum Brechen von Licht vom Auge (10) ist, das vom drehbaren Spiegel (30) am ersten Brennpunkt (32-1) des ellipsoidalen Spiegels (32) reflektiert wurde, um sich entlang der ersten optischen Achse (48-1) und der zweiten optischen Achse (48-2) auszubreiten.

7. Ophthalmisches Ultraweitfeld-Bildgebungsinstrument (100) nach einem der Ansprüche 1 bis 4, wobei die Stereobildgebungsvorrichtung (40) umfasst:
eine erste Kamera (42) mit einer ersten optischen Achse (48-1) und eine zweite Kamera (44) mit einer zweiten optischen Achse (48-2), die parallel zur ersten optischen Achse (48-1) ist; und
eine Fresnel-Linse (49), die zwischen dem drehbaren Spiegel (30) und sowohl der ersten Kamera (42) als auch der zweiten Kamera (44) angeordnet ist, wobei die Fresnel-Linse (49) zum Brechen von Licht vom Auge (10), das vom drehbaren Spiegel (30) reflektiert wurde, um sich entlang der ersten optischen Achse (48-1) und der zweiten optischen Achse (48-2) auszubreiten eingerichtet ist.

8. Ophthalmisches Ultraweitfeld-Bildgebungsinstrument (100) nach einem der vorhergehenden Ansprüche, ferner umfassend:
eine Fixierziel-Lichtquelle (80), eingerichtet zum Projizieren von Fixierlicht (LF) auf den Fundus (20) mittels des drehbaren Spiegels (30) und des ellipsoidalen Spiegels (32),
wobei im Patientenausrichtungsmodus der Prozessor (50) zum Steuern der Fixierziel-Lichtquelle (80) zum Projizieren des Fixierlichts (LF) auf den drehbaren Spiegel (30) in der vorbestimmten Orientierung eingerichtet ist, wobei die Fixierziel-Lichtquelle (80) in Bezug auf den drehbaren Spiegel (30) derart angeordnet ist, dass das Fixierlicht (LF) mittels des ellipsoidalen Spiegels (32) auf den Fundus (12) projiziert wird, um eine Blickrichtung des Auges (10) zu fixieren.

9. Ophthalmisches Ultraweitfeld-Bildgebungsinstrument (100) nach Anspruch 8, wenn abhängig von Anspruch 2, wobei die Fixierziel-Lichtquelle (80) innerhalb des Bereichs (R) zwischen dem drehbaren Spiegel (30) und dem ellipsoidalen Spiegel (32) angeordnet ist, der während der Erfassung des Ultraweitfeld-Bildes (55) des Fundus (12) nicht vom Lichtstrahl (LT) durchquert wird.

10. Ophthalmisches Ultraweitfeld-Bildgebungsinstrument (100) nach Anspruch 9, wobei die vorbestimmte Orientierung derart ist, dass der drehbare Spiegel (30) beginnt, sich aus der vorbestimmten Orientierung zu drehen, wenn das ophthalmische Ultraweitfeld-Bildgebungsinstrument (100) beginnt, das Ultraweitfeld-Bild (55) des Fundus (12) zu erfassen.

11. Ophthalmisches Ultraweitfeld-Bildgebungsinstrument (100) nach Anspruch 10, wobei die Fixierziel-Lichtquelle (80) eingerichtet zum Projizieren des Fixierlichts (LF) auf den drehbaren Spiegel (30) von einer Position derart ist, dass, wenn der drehbare Spiegel (30) in der vorbestimmten Orientierung ist, das Fixierlicht (LF) in einer Richtung zur zentralen Blickfixierung des Auges (10) auf das Auge (10) einfällt.

12. Ophthalmisches Ultraweitfeld-Bildgebungsinstrument (100) nach einem der vorhergehenden Ansprüche, ferner umfassend eine Beleuchtungsquelle (47), eingerichtet zum Beleuchten des Auges (10) mittels des drehbaren Spiegels (30) und des ellipsoidalen Spiegels (32) mit Licht zum Erfassen der Stereobilder (46-1; 46-2) der Pupille (14) durch die Stereobildgebungsvorrichtung (40).

13. Ophthalmisches Ultraweitfeld-Bildgebungsinstrument (100) nach einem der vorhergehenden Ansprüche, wobei das ophthalmische Ultraweitfeld-Bildgebungsinstrument (100) ein Ultraweitfeld-Abtastlaser-Ophthalmoskop umfasst.

14. Verfahren zum Steuern eines ophthalmischen Ultraweitfeld-Bildgebungsinstruments (100; 300) zum Erfassen eines Ultraweitfeld-Bildes (55) eines Fundus (12) eines Auges (10) durch Abtasten eines Lichtstrahls (LT; LT') über den Fundus (12), wobei das ophthalmische Ultraweitfeld-Bildgebungsinstrument (100) umfasst:
eine Lichtquelle (20; 20'), eingerichtet zum Emittieren des Lichtstrahls (LT; LT');
einen drehbaren Spiegel (30), eingerichtet zum Abtasten des Lichtstrahls (LT; LT') über den Fundus (12);
einen ellipsoidalen Spiegel (32), mittels dessen der drehbare Spiegel (30) zum Abtasten des Lichtstrahls (LT; LT') über den Fundus (12) eingerichtet ist, wobei der ellipsoidale Spiegel (32) einen ersten Brennpunkt (32-1) und einen zweiten Brennpunkt (32-2) aufweist, wobei der drehbare Spiegel (30) zum Abtasten des Lichtstrahls (LT; LT') über den ellipsoidalen Spiegel (32) durch den ersten Brennpunkt (32-1) eingerichtet ist und eine Pupille (14) des Auges (10) während der Verwendung des ophthalmischen Ultraweitfeld-Bildgebungsinstruments (100) am zweiten Brennpunkt (32-2) positioniert ist; und
eine Stereobildgebungsvorrichtung (40), eingerichtet zum Erfassen von Stereobildern (46-1; 46-2) der Pupille (14) mittels des drehbaren Spiegels (30) und des ellipsoidalen Spiegels (32),
wobei das Verfahren umfasst:
Erzeugen eines Signals (S) zum Bringen der Pupille (14) des Auges (10) in einen Zielbereich zum Erfassen des Ultraweitfeld-Bildes (55) des Fundus (12), durch:
Steuern (S10) des drehbaren Spiegels (30), feststehend in einer vorbestimmten Orientierung zu sein, die es der Stereobildgebungsvorrichtung (40) ermöglicht, die Pupille (14) mittels des drehbaren Spiegels (30) und des ellipsoidalen Spiegels (32) abzubilden;
Steuern (S20) der Stereobildgebungsvorrichtung (40) zum Erfassen der Stereobilder (46-1; 46-2) der Pupille (14) mittels des drehbaren Spiegels (30) und des ellipsoidalen Spiegels (32), während der drehbare Spiegel (30) in der vorbestimmten Orientierung ist; und
Verarbeiten (S30) der Stereobilder (46-1; 46-2) der Pupille (14) zum Erzeugen des Signals (S); und
nach dem Erzeugen des Signals (S), Steuern (S40) der Lichtquelle (20; 20') zum Emittieren des Lichtstrahls (LT; LT') und Steuern des ophthalmischen Ultraweitfeld-Bildgebungsinstruments (100) zum Erfassen des Ultraweitfeld-Bildes (55) des Fundus (12) durch Drehen des drehbaren Spiegels (30) zum Abtasten des Lichtstrahls (LT; LT') über den Fundus (12) mittels des ellipsoidalen Spiegels (32).

15. Verfahren nach Anspruch 14, wobei
das ophthalmische Ultraweitfeld-Bildgebungsinstrument (100) ferner eine Fixierziel-Lichtquelle (80) umfasst, die zum Projizieren von Fixierlicht (LF) auf den Fundus (20) mittels des drehbaren Spiegels (30) und des ellipsoidalen Spiegels (32) eingerichtet ist, und
das Verfahren ferner umfasst:
während der drehbare Spiegel (30) in der vorbestimmten Orientierung ist und die Stereobildgebungsvorrichtung (40) zum Erfassen der Stereobilder (46-1; 46-2) der Pupille (14) gesteuert wird, Steuern der Fixierziel-Lichtquelle (80) zum Projizieren des Fixierlichts (LF) auf den drehbaren Spiegel (30), wobei die Fixierziel-Lichtquelle (80) in Bezug auf den drehbaren Spiegel (30) derart angeordnet ist, dass das Fixierlicht (LF) mittels des ellipsoidalen Spiegels (32) auf den Fundus (12) projiziert wird, um eine Blickrichtung des Auges (10) zu fixieren.

## Revendications

1. Instrument d'imagerie ophtalmique à champ ultra-large (100 ; 300) agencé pour acquérir une image à champ ultra-large (55) d'un fond de l'œil (12) d'un œil (10) en balayant le fond de l'œil (12) avec un faisceau lumineux (LT), comprenant :
une source lumineuse (20 ; 20') agencée pour émettre le faisceau lumineux (L_{T}; L_{T}') ;
un miroir rotatif (30) agencé pour balayer le faisceau lumineux (L_{T}; L_{T}') sur le fond de l'œil (12) ;
un miroir ellipsoïdal (32) par l'intermédiaire duquel le miroir rotatif (30) est agencé pour balayer le faisceau lumineux (L_{T}; L_{T}') sur le fond de l'œil (12), le miroir ellipsoïdal (32) ayant un premier point focal (32-1) et un deuxième point focal (32-2), dans lequel le miroir rotatif (30) est agencé pour balayer le faisceau lumineux (L_{T}; L_{T}') à travers le miroir ellipsoïdal (32) via le premier point focal (32-1), et une pupille (14) de l'œil (10) est positionnée au niveau du deuxième point focal (32-2) lors d'une utilisation de l'instrument d'imagerie ophtalmique à champ ultra-large (100) ;
un appareil d'imagerie stéréoscopique (40) agencé pour acquérir des images stéréoscopiques (46-1 ; 46-2) de la pupille (14) via le miroir rotatif (30) et le miroir ellipsoïdal (32) ; et
un processeur (50) agencé pour fonctionner en un mode d'alignement de patient afin de générer, sur la base des images stéréoscopiques (46-1 ; 46-2) de la pupille (14), un signal (S) pour amener la pupille (14) de l'œil (10) dans une plage cible pour acquérir une image à champ ultra-large (55) du fond de l'œil (12), puis de fonctionner en un mode d'imagerie de fond de l'œil afin de commander l'instrument d'imagerie ophtalmique à champ ultra-large (100) pour acquérir l'image à champ ultra-large (55) du fond de l'œil (12), dans lequel
en mode d'alignement de patient, le processeur (50) est agencé pour :
commander le miroir rotatif (30) afin qu'il reste immobile dans une orientation prédéterminée ;
commander l'appareil d'imagerie stéréoscopique (40) pour acquérir les images stéréoscopiques (46-1 ; 46-2) de la pupille (14) via le miroir rotatif (30) et le miroir ellipsoïdal (32) tandis que le miroir rotatif (30) est immobile dans l'orientation prédéterminée ; et
traiter les images stéréoscopiques (46-1 ; 46-2) de la pupille (14) afin de générer le signal (S) pour aligner la pupille (14) de l'œil (10) avec la position d'imagerie ; et
en mode d'imagerie de fond de l'œil, le processeur (50) est agencé pour :
commander la source lumineuse (20 ; 20') pour émettre le faisceau lumineux (L_{T}; L_{T}') ; et
commander l'instrument d'imagerie ophtalmique à champ ultra-large (100 ; 300) pour acquérir l'image à champ ultra-large (55) du fond de l'œil (12) en commandant le miroir rotatif (30) pour balayer le faisceau lumineux (L_{T}; L_{T}') sur le fond de l'œil (12) via le miroir ellipsoïdal (32).

2. Instrument d'imagerie ophtalmique à champ ultra-large (100) selon la revendication 1, dans lequel le miroir rotatif (30) est agencé pour réfléchir le faisceau lumineux (L_{T}) au premier point focal (32-1), et l'appareil d'imagerie stéréoscopique (40) est situé dans une région (R) entre le miroir rotatif (30) et le miroir ellipsoïdal (32) qui n'est pas traversée par le faisceau lumineux (L_{T}) pendant une acquisition de l'image à champ ultra-large (55) du fond de l'œil (12).

3. Instrument d'imagerie ophtalmique à champ ultra-large (100) selon la revendication 2, dans lequel l'orientation prédéterminée du miroir rotatif (30) est telle que le miroir rotatif (30) commence à tourner à partir de l'orientation prédéterminée lorsque l'instrument d'imagerie ophtalmique à champ ultra-large (100) commence à acquérir l'image à champ ultra-large (55) du fond de l'œil (12).

4. Instrument d'imagerie ophtalmique à champ ultra-large (100) selon la revendication 2 ou la revendication 3, dans lequel l'appareil d'imagerie stéréoscopique (40) est situé entre le miroir rotatif (30) et le miroir ellipsoïdal (32) à un emplacement qui minimise un écart par rapport à une circularité des représentations respectives de la pupille (14) dans les images stéréoscopiques (46-1 ; 46-2) de la pupille (14) acquises par l'appareil d'imagerie stéréoscopique (40) lorsque le miroir rotatif (30) se trouve dans l'orientation prédéterminée.

5. Instrument d'imagerie ophtalmique à champ ultra-large (100) selon la revendication 4, dans lequel
le miroir ellipsoïdal (32) a un plan de symétrie (P) comprenant le premier point focal (32-1) et le deuxième point focal (32-2),
une direction normale du miroir rotatif (30) au niveau du premier point focal (32-1) sous-tend un angle prédéterminé par rapport au plan de symétrie (P) lorsque le miroir rotatif (30) se trouve dans l'orientation prédéterminée, et
l'appareil d'imagerie stéréoscopique (40) comprend une première caméra (42) et une deuxième caméra (44), dans lequel la première caméra (42) et la deuxième caméra (44) sont disposées à égale distance et de part et d'autre d'un deuxième plan (P2) comprenant le premier point focal (32-1) et le deuxième point focal (32-2), la direction normale du miroir rotatif (30) et du deuxième plan (P2) formant, au niveau du premier point focal (32-1), un angle inférieur à deux fois l'angle prédéterminé.

6. Instrument d'imagerie ophtalmique à champ ultra-large (100) selon la revendication 5, dans lequel la première caméra (42) a un premier axe optique (48-1), et la deuxième caméra (44) a un deuxième axe optique (48-2) qui est parallèle au premier axe optique (48-1), et l'appareil d'imagerie stéréoscopique (40) comprend en outre une lentille de Fresnel (49) disposée entre le miroir rotatif (30) et à la fois la première caméra (42) et la deuxième caméra (44), la lentille de Fresnel (49) étant agencée pour réfracter de la lumière provenant de l'œil (10), qui a été réfléchie par le miroir rotatif (30) au niveau du premier point focal (32-1) du miroir ellipsoïdal (32), pour se propager le long du premier axe optique (48-1) et du deuxième axe optique (48-2).

7. Instrument d'imagerie ophtalmique à champ ultra-large (100) selon l'une quelconque des revendications 1 à 4, dans lequel l'appareil d'imagerie stéréoscopique (40) comprend :
une première caméra (42) ayant un premier axe optique (48-1), et une deuxième caméra (44) ayant un deuxième axe optique (48-2) qui est parallèle au premier axe optique (48-1) ; et
une lentille de Fresnel (49) disposée entre le miroir rotatif (30) et à la fois la première caméra (42) et la deuxième caméra (44), la lentille de Fresnel (49) étant agencée pour réfracter de la lumière provenant de l'œil (10), qui a été réfléchie par le miroir rotatif (30), pour se propager le long du premier axe optique (48-1) et du deuxième axe optique (48-2).

8. Instrument d'imagerie ophtalmique à champ ultra-large (100) selon l'une quelconque des revendications précédentes, comprenant en outre :
une source lumineuse de cible de fixation (80) agencée pour projeter une lumière de fixation (L_{F}) sur le fond de l'œil (20) via le miroir rotatif (30) et le miroir ellipsoïdal (32),
dans lequel, dans le mode d'alignement de patient, le processeur (50) est agencé pour commander la source lumineuse de cible de fixation (80) pour projeter la lumière de fixation (L_{F}) sur le miroir rotatif (30) dans l'orientation prédéterminée, la source lumineuse de cible de fixation (80) étant disposée par rapport au miroir rotatif (30) de telle sorte que la lumière de fixation (L_{F}) soit projetée sur le fond de l'œil (12) via le miroir ellipsoïdal (32) afin de fixer une direction de regard de l'œil (10).

9. Instrument d'imagerie ophtalmique à champ ultra-large (100) selon la revendication 8, lorsqu'elle dépend de la revendication 2, dans lequel la source lumineuse de cible de fixation (80) est située dans la région (R) entre le miroir rotatif (30) et le miroir ellipsoïdal (32) qui n'est pas traversée par le faisceau lumineux (L_{T}) pendant une acquisition de l'image à champ ultra-large (55) du fond de l'œil (12).

10. Instrument d'imagerie ophtalmique à champ ultra-large (100) selon la revendication 9, dans lequel l'orientation prédéterminée est telle que le miroir rotatif (30) commence à tourner à partir de l'orientation prédéterminée lorsque l'instrument d'imagerie ophtalmique à champ ultra-large (100) commence à acquérir l'image à champ ultra-large (55) du fond de l'œil (12).

11. Instrument d'imagerie ophtalmique à champ ultra-large (100) selon la revendication 10, dans lequel la source lumineuse de cible de fixation (80) est agencée pour projeter la lumière de fixation (L_{F}) sur le miroir rotatif (30) depuis un emplacement tel que, lorsque le miroir rotatif (30) se trouve dans l'orientation prédéterminée, la lumière de fixation (L_{F}) soit incidente sur l'œil (10) dans une direction pour une fixation centrale de regard de l'œil (10).

12. Instrument d'imagerie ophtalmique à champ ultra-large (100) selon l'une quelconque des revendications précédentes, comprenant en outre une source d'éclairage (47) agencée pour éclairer l'œil (10) via le miroir rotatif (30) et le miroir ellipsoïdal (32) avec de la lumière pour acquérir les images stéréoscopiques (46-1 ; 46-2) de la pupille (14) par l'appareil d'imagerie stéréoscopique (40).

13. Instrument d'imagerie ophtalmique à champ ultra-large (100) selon l'une quelconque des revendications précédentes, dans lequel l'instrument d'imagerie ophtalmique à champ ultra-large (100) comprend un ophtalmoscope laser à balayage à champ ultra-large.

14. Procédé de commande d'un instrument d'imagerie ophtalmique à champ ultra-large (100 ; 300) pour acquérir une image à champ ultra-large (55) d'un fond de l'œil (12) d'un œil (10) en balayant un faisceau lumineux (L_{T}; L_{T}') sur le fond de l'œil (12), l'instrument d'imagerie ophtalmique à champ ultra-large (100) comprenant :
une source lumineuse (20 ; 20') agencée pour émettre le faisceau lumineux (L_{T}; L_{T}') ;
un miroir rotatif (30) agencé pour balayer le faisceau lumineux (L_{T}; L_{T}') sur le fond de l'œil (12) ;
un miroir ellipsoïdal (32) par l'intermédiaire duquel le miroir rotatif (30) est agencé pour balayer le faisceau lumineux (L_{T}; L_{T}') sur le fond de l'œil (12), le miroir ellipsoïdal (32) ayant un premier point focal (32-1) et un deuxième point focal (32-2), dans lequel le miroir rotatif (30) est agencé pour balayer le faisceau lumineux (L_{T}; L_{T}') à travers le miroir ellipsoïdal (32) via le premier point focal (32-1) et une pupille (14) de l'œil (10) est positionnée au niveau du deuxième point focal (32-2) lors d'une utilisation de l'instrument d'imagerie ophtalmique à champ ultra-large (100) ; et
un appareil d'imagerie stéréoscopique (40) agencé pour acquérir des images stéréoscopiques (46-1 ; 46-2) de la pupille (14) via le miroir rotatif (30) et le miroir ellipsoïdal (32),
le procédé comprenant :
la génération d'un signal (S) pour amener la pupille (14) de l'œil (10) dans une plage cible pour acquérir l'image à champ ultra-large (55) du fond de l'œil (12), en :
commandant (S10) le miroir rotatif (30) afin qu'il reste immobile dans une orientation prédéterminée qui permet à l'appareil d'imagerie stéréoscopique (40) de former une image de la pupille (14) via le miroir rotatif (30) et le miroir ellipsoïdal (32) ;
commandant (S20) l'appareil d'imagerie stéréoscopique (40) pour acquérir les images stéréoscopiques (46-1 ; 46-2) de la pupille (14) via le miroir rotatif (30) et le miroir ellipsoïdal (32) pendant que le miroir rotatif (30) se trouve dans l'orientation prédéterminée ; et
traitant (S30) les images stéréoscopiques (46-1 ; 46-2) de la pupille (14) afin de générer le signal (S) ; et
après avoir généré le signal (S), la commande (S40) de la source lumineuse (20 ; 20') pour émettre le faisceau lumineux (L_{T}; L_{T}'), et la commande de l'instrument d'imagerie ophtalmique à champ ultra-large (100) pour acquérir l'image à champ ultra-large (55) du fond de l'œil (12) en faisant tourner le miroir rotatif (30) pour balayer le faisceau lumineux (L_{T}; L_{T}') sur le fond de l'œil (12) via le miroir ellipsoïdal (32).

15. Procédé selon la revendication 14, dans lequel
l'instrument d'imagerie ophtalmique à champ ultra-large (100) comprend en outre une source lumineuse de cible de fixation (80) agencée pour projeter une lumière de fixation (L_{F}) sur le fond de l'œil (12) via le miroir rotatif (30) et le miroir ellipsoïdal (32), et
le procédé comprend en outre :
alors que le miroir rotatif (30) se trouve dans l'orientation prédéterminée et que l'appareil d'imagerie stéréoscopique (40) est commandé pour acquérir les images stéréoscopiques (46-1 ; 46-2) de la pupille (14), la commande de la source lumineuse de cible de fixation (80) pour projeter la lumière de fixation (L_{F}) sur le miroir rotatif (30), la source lumineuse de cible de fixation (80) étant disposée par rapport au miroir rotatif (30) de telle sorte que la lumière de fixation (L_{F}) soit projetée sur le fond de l'œil (12) via le miroir ellipsoïdal (32) afin de fixer une direction de regard de l'œil (10).
